# EUROPÄISCHE PATENTANMELDUNG

(11) **EP 1 932 922 A1**
(43) Veröffentlichungstag der Anmeldung: **18.06.2008**
(21) Anmeldenummer: 06400039.1
(22) Anmeldetag: 13.12.2006
(51) Int. Cl.: C12Q 1/68

(54) **Schnelltest zum Nachweis von DNA-Sequenzen**

(71) Anmelder: Desitin Arzneimittel GmbH, D-22335 Hamburg (DE)
(72) Erfinder: Peitz, Dieter, 22297 Hamburg (DE); Zentgraf, Martin, 22085 Hamburg (DE)
(74) Vertreter: UEXKÜLL & STOLBERG

(57) **Zusammenfassung**

Die vorliegende Erfindung betrifft in vitro-Verfahren zum Nachweis einer DNA-Zielsequenz in Zellen unter Verwendung eines Oligonukleotids, das mit einem beta-D-Galactopyranosid markiert ist, welches bei Hydrolyse der glycosidischen Bindung einen wasserunlöslichen Farbstoff bildet. Die Erfindung betrifft insbesondere ein in vitro-Verfahren zum Nachweis einer DNA-Zielsequenz aus einer Gruppe von DNA-Sequenzen, deren Mitglieder sich in genau einer vorbestimmten Nukleotid-Position voneinander unterscheiden, unter Verwendung eines Oligonukleotids, das mit einem beta-D-Galactopyranosid markiert ist, welches bei Hydrolyse der glycosidischen Bindung einen wasserunlöslichen Farbstoff bildet. Die Erfindung betrifft außerdem die Verwendung eines solchen Oligonukleotids in DNA-Hybridisierungsverfahren. Schließlich betrifft die Erfindung Kits zur Durchführung der obigen Verfahren.

SEQUENZPROTOKOLL

<110>  Desitin Arzneimittel GmbH

<120>  Schnelltest zum Nachweis von DNA-Sequenzen

<130>  P 72380

<160>  5

<170>  PatentIn version 3.3

<210>  1
<211>  1024
<212>  PRT
<213>  Escherichia coli

<220>
<223>  beta-Galactosidase

<400>  1
Met Thr Met Ile Thr Asp Ser Leu Ala Val Val Leu Gln Arg Arg Asp
1               5                   10                  15

Trp Glu Asn Pro Gly Val Thr Gln Leu Asn Arg Leu Ala Ala His Pro
            20                  25                  30

Pro Phe Ala Ser Trp Arg Asn Ser Glu Glu Ala Arg Thr Asp Arg Pro
        35                  40                  45

Ser Gln Gln Leu Arg Ser Leu Asn Gly Glu Trp Arg Phe Ala Trp Phe
    50                  55                  60

Pro Ala Pro Glu Ala Val Pro Glu Ser Trp Leu Glu Cys Asp Leu Pro
65                  70                  75                  80

Glu Ala Asp Thr Val Val Val Pro Ser Asn Trp Gln Met His Gly Tyr
                85                  90                  95

Asp Ala Pro Ile Tyr Thr Asn Val Thr Tyr Pro Ile Thr Val Asn Pro
            100                 105                 110

Pro Phe Val Pro Thr Glu Asn Pro Thr Gly Cys Tyr Ser Leu Thr Phe
        115                 120                 125

Asn Val Asp Glu Ser Trp Leu Gln Glu Gly Gln Thr Arg Ile Ile Phe
    130                 135                 140

Asp Gly Val Asn Ser Ala Phe His Leu Trp Cys Asn Gly Arg Trp Val
145                 150                 155                 160

Gly Tyr Gly Gln Asp Ser Arg Leu Pro Ser Glu Phe Asp Leu Ser Ala
                165                 170                 175
Phe Leu Arg Ala Gly Glu Asn Arg Leu Ala Val Met Val Leu Arg Trp
            180                 185                 190

Ser Asp Gly Ser Tyr Leu Glu Asp Gln Asp Met Trp Arg Met Ser Gly
        195                 200                 205

Ile Phe Arg Asp Val Ser Leu Leu His Lys Pro Thr Thr Gln Ile Ser
    210                 215                 220

Asp Phe His Val Ala Thr Arg Phe Asn Asp Asp Phe Ser Arg Ala Val
225                 230                 235                 240

Leu Glu Ala Glu Val Gln Met Cys Gly Glu Leu Arg Asp Tyr Leu Arg
                245                 250                 255

Val Thr Val Ser Leu Trp Gln Gly Glu Thr Gln Val Ala Ser Gly Thr
            260                 265                 270

Ala Pro Phe Gly Gly Glu Ile Ile Asp Glu Arg Gly Gly Tyr Ala Asp
        275                 280                 285

Arg Val Thr Leu Arg Leu Asn Val Glu Asn Pro Lys Leu Trp Ser Ala
    290                 295                 300

Glu Ile Pro Asn Leu Tyr Arg Ala Val Val Glu Leu His Thr Ala Asp
305                 310                 315                 320

Gly Thr Leu Ile Glu Ala Glu Ala Cys Asp Val Gly Phe Arg Glu Val
                325                 330                 335

Arg Ile Glu Asn Gly Leu Leu Leu Leu Asn Gly Lys Pro Leu Leu Ile
            340                 345                 350

Arg Gly Val Asn Arg His Glu His His Pro Leu His Gly Gln Val Met
        355                 360                 365

Asp Glu Gln Thr Met Val Gln Asp Ile Leu Leu Met Lys Gln Asn Asn
    370                 375                 380

Phe Asn Ala Val Arg Cys Ser His Tyr Pro Asn His Pro Leu Trp Tyr
385                 390                 395                 400

Thr Leu Cys Asp Arg Tyr Gly Leu Tyr Val Val Asp Glu Ala Asn Ile
                405                 410                 415

Glu Thr His Gly Met Val Pro Met Asn Arg Leu Thr Asp Asp Pro Arg
            420                 425                 430

Trp Leu Pro Ala Met Ser Glu Arg Val Thr Arg Met Val Gln Arg Asp
        435                 440                 445

Arg Asn His Pro Ser Val Ile Ile Trp Ser Leu Gly Asn Glu Ser Gly
    450                 455                 460

His Gly Ala Asn His Asp Ala Leu Tyr Arg Trp Ile Lys Ser Val Asp
465                 470                 475                 480

Pro Ser Arg Pro Val Gln Tyr Glu Gly Gly Gly Ala Asp Thr Thr Ala
                485                 490                 495

Thr Asp Ile Ile Cys Pro Met Tyr Ala Arg Val Asp Glu Asp Gln Pro
            500                 505                 510

Phe Pro Ala Val Pro Lys Trp Ser Ile Lys Lys Trp Leu Ser Leu Pro
        515                 520                 525

Gly Glu Thr Arg Pro Leu Ile Leu Cys Glu Tyr Ala His Ala Met Gly
    530                 535                 540

Asn Ser Leu Gly Gly Phe Ala Lys Tyr Trp Gln Ala Phe Arg Gln Tyr
545                 550                 555                 560

Pro Arg Leu Gln Gly Gly Phe Val Trp Asp Trp Val Asp Gln Ser Leu
                565                 570                 575

Ile Lys Tyr Asp Glu Asn Gly Asn Pro Trp Ser Ala Tyr Gly Gly Asp
            580                 585                 590

Phe Gly Asp Thr Pro Asn Asp Arg Gln Phe Cys Met Asn Gly Leu Val
        595                 600                 605

Phe Ala Asp Arg Thr Pro His Pro Ala Leu Thr Glu Ala Lys His Gln
    610                 615                 620

Gln Gln Phe Phe Gln Phe Arg Leu Ser Gly Gln Thr Ile Glu Val Thr
625                 630                 635                 640

Ser Glu Tyr Leu Phe Arg His Ser Asp Asn Glu Leu Leu His Trp Met
                645                 650                 655

Val Ala Leu Asp Gly Lys Pro Leu Ala Ser Gly Glu Val Pro Leu Asp
            660                 665                 670

Val Ala Pro Gln Gly Lys Gln Leu Ile Glu Leu Pro Glu Leu Pro Gln
        675                 680                 685

Pro Glu Ser Ala Gly Gln Leu Trp Leu Thr Val Arg Val Val Gln Pro
    690                 695                 700

Asn Ala Thr Ala Trp Ser Glu Ala Gly His Ile Ser Ala Trp Gln Gln
705                 710                 715                 720

Trp Arg Leu Ala Glu Asn Leu Ser Val Thr Leu Pro Ala Ala Ser His
                725                 730                 735

Ala Ile Pro His Leu Thr Thr Ser Glu Met Asp Phe Cys Ile Glu Leu
            740                 745                 750

Gly Asn Lys Arg Trp Gln Phe Asn Arg Gln Ser Gly Phe Leu Ser Gln
        755                 760                 765

Met Trp Ile Gly Asp Lys Lys Gln Leu Leu Thr Pro Leu Arg Asp Gln
    770                 775                 780

Phe Thr Arg Ala Pro Leu Asp Asn Asp Ile Gly Val Ser Glu Ala Thr
785                 790                 795                 800

Arg Ile Asp Pro Asn Ala Trp Val Glu Arg Trp Lys Ala Ala Gly His
                805                 810                 815

Tyr Gln Ala Glu Ala Ala Leu Leu Gln Cys Thr Ala Asp Thr Leu Ala
            820                 825                 830

Asp Ala Val Leu Ile Thr Thr Ala His Ala Trp Gln His Gln Gly Lys
        835                 840                 845

Thr Leu Phe Ile Ser Arg Lys Thr Tyr Arg Ile Asp Gly Ser Gly Gln
    850                 855                 860

Met Ala Ile Thr Val Asp Val Glu Val Ala Ser Asp Thr Pro His Pro
865                 870                 875                 880

Ala Arg Ile Gly Leu Asn Cys Gln Leu Ala Gln Val Ala Glu Arg Val
                885                 890                 895

Asn Trp Leu Gly Leu Gly Pro Gln Glu Asn Tyr Pro Asp Arg Leu Thr
            900                 905                 910

Ala Ala Cys Phe Asp Arg Trp Asp Leu Pro Leu Ser Asp Met Tyr Thr
        915                 920                 925

Pro Tyr Val Phe Pro Ser Glu Asn Gly Leu Arg Cys Gly Thr Arg Glu
    930                 935                 940

Leu Asn Tyr Gly Pro His Gln Trp Arg Gly Asp Phe Gln Phe Asn Ile
945                 950                 955                 960

Ser Arg Tyr Ser Gln Gln Gln Leu Met Glu Thr Ser His Arg His Leu
                965                 970                 975

Leu His Ala Glu Glu Gly Thr Trp Leu Asn Ile Asp Gly Phe His Met
            980                 985                 990

Gly Ile Gly Gly Asp Asp Ser Trp Ser Pro Ser Val Ser Ala Glu Phe
        995                 1000                1005

Gln Leu Ser Ala Gly Arg Tyr His Tyr Gln Leu Val Trp Cys Gln
    1010                1015                1020

Lys

<210> 2
<211> 2322
<212> DNA
<213> Staphylococcus aureus

<220>
<223> mecA-Gen

<400> 2
atgaaaaaga taaaaattgt tccacttatt ttaatagttg tagttgtcgg gtttggtata 60

tatttttatg cttcaaaaga taaagaaatt aataatacta ttgatgcaat tgaagataaa 120

aatttcaaac aagtttataa agatagcagt tatatttcta aaagcgataa tggtgaagta 180

gaaatgactg aacgtccgat aaaaatatat aatagtttag gcgttaaaga tataaacatt 240

caggatcgta aaataaaaaa agtatctaaa aataaaaaac gagtagatgc tcaatataaa 300

attaaaacaa actacggtaa cattgatcgc aacgttcaat ttaattttgt taaagaagat 360

ggtatgtgga agttagattg ggatcatagc gtcattattc caggaatgca gaaagaccaa 420

agcatacata ttgaaaattt aaaatcagaa cgtggtaaaa ttttagaccg aaacaatgtg 480

gaattggcca atacaggaac acatatgaga ttaggcatcg ttccaaagaa tgtatctaaa 540

aaagattata aagcaatcgc taaagaacta agtatttctg aagactatat caacaacaaa 600

tggatcaaaa ttgggtacaa gatgatacct tcgttccact ttaaaaccgt taaaaaaatg 660

gatgaatatt taagtgattt cgcaaaaaaa tttcatctta caactaatga aacagaaagt 720

cgtaactatc ctctaggaaa agcgacttca catctattag gttatgttgg tcccattaac 780

tctgaagaat taaaacaaaa agaatataaa ggctataaag atgatgcagt tattggtaaa 840

aagggactcg aaaaacttta cgataaaaag ctccaacatg aagatggcta tcgtgtcaca 900

atcgttagag tcgacgataa tagcaataca atcgcacata cattaataga gaaaaagaaa 960

aaagatggca aagatattca actaactatt gatgctaaag ttcaaaagag tatttataac 1020

aacatgaaaa atgattatgg ctcaggtact gctatccacc ctcaaacagg tgaattatta 1080

gcacttgtaa gcacaccttc atatgacgtc tatccattta tgtatggcat gagtaacgaa 1140

gaatataata aattaaccga agataaaaaa gaacctctgc tcaacaagtt ccagattaca 1200

acttcaccag gttcaactca aaaaatatta acagcaatga ttgggttaaa taacaaaaca 1260
ttagacgata aaacaagtta taaaatcgat ggtaaaggtt ggcaaaaaga taaatcttgg 1320
ggtggttaca acgttacaag atatgaagtg gtaaatggta atatcgactt aaaacaagca 1380
atagaatcat cagataacat tttctttgct agagtagcac tcgaattagg cagtaagaaa 1440
tttgaaaaag gcatgaaaaa actaggtgtt ggtgaagata taccaagtga ttatccattt 1500
tataatgctc aaatttcaaa caaaaattta gataatgaaa tattattagc tgattcaggt 1560
tacggacaag gtgaaatact gattaaccca gtacagatcc tttcaatcta tagcgcatta 1620
gaaaataatg gcaatattaa cgcacctcac ttattaaaag acacgaaaaa caaagtttgg 1680
aagaaaaata ttatttccaa agaaaatatc aatctattaa atgatggtat gcaacaagtc 1740
gtaaataaaa cacataaaga agatatttat agatcttatg caaacttaat tggcaaatcc 1800
ggtactgcag aactcaaaat gaaacaagga gaaactggca gacaaattgg gtggtttata 1860
tcatatgata aagataatcc aaacatgatg atggctatta atgttaaaga tgtacaagat 1920
aaaggaatgg ctagctacaa tgccaaaatc tcaggtaaag tgtatgatga gctatatgag 1980
aacggtaata aaaaatacga tatagatgaa taacaaaagc agtgaagcat ccgtaacgat 2040
ggttgcttca ctgttttatt atgaattatt aataagtgct gttacttctc cttaaataca 2100
tttctcattt catgtatgtt gaaagtgaca ctgtaacgag tccattttct ttttttatgg 2160
atttcttatt tgtaatttca gcgataacgt acaatgtatt acctgggtat acaggtttaa 2220
taaattttaa cgttattcat gttgtgttcc tgctacaact tcttctccgt atttaccttc 2280
ttctacccat aatttaaatg atattgaaag tgtattgcat gc 2322

<210> 3
<211> 14396
<212> DNA
<213> Homo sapiens

<220>
<223> SV2A-Gen

<400> 3
ttctccccgt gccctgactc cctccccatc ctcttttctc ctctccccac caatggtctc 60
tcccgatgct cagtggcgcg gggtaggggg gggcggaggc ggggctggag tggtggaagg 120
ggggtggcag gtctgcattg ccgcttccct ggtgccggga gcagtcgccg ctgccgcctc 180
cgcccgcggc cgggaccccc gtcctcgccc gggactcctt acccggggaa cctagaccag 240
gtgagacccc tttctcagtc gctcacctcc tccccgcccc tggcagaagc atctttgcct 300
tgccttgcag ccattttatc ctccgatgct ttgcttatgg ggagggtgac gatcccctct 360
ctaggggtga ggggggttgg tgggttggtg gtggtttggg gatggacatt tatttcagtt 420
gtgctcttca ggacacacac acacacacgc atatgtataa tattggtgaa tggggaggag 480
gggtgttttt ctctacctta accaagtccc cactatcccc ttcggagatt aacgtggtgg 540

tggggttgtg aggtgggatg aggttccctc aaactcctat cccctcctca ccttgacgtc 600
cccccacccc caggatgaat gataaatgac cttggctggg gctggagaat ggatgctgcc 660
tcagtgtgca cgcctggact tgggtgggtg gggggcaggg gaagaagggc aggtgaagga 720
tggagggaag gggagtgaag aatggaaata ctggggagac agagatgtag agccgccagg 780
tgttgggggc atggatggcg gggctggcg gcagttgctc cggggtgggg ggaagggatg 840
ttgagtgaag gtggacactt gtctagcagc aggaccaagg gtggaggagg tgctcagagc 900
agggatttag tcagttctgg gggcttttcc cttattccgg agctgaggca agatggggtg 960
tgggggggtc gctgcaaatg ctgatagggg cggtctctat gtctgtgcat gcggaggtct 1020
ctccctgcgt ccccctctct gcctctccgt attcctcagt gtgcattctc agctgacacc 1080
ctcattttta tccctcagaa ctgtgggtgc tcctatcttc ctaaccttgg taggatgtgg 1140
ggaggggagg cgcagtcttg atggtcctgg gtgggggctg aagttcgatg accgggctgt 1200
catggggaag ggtgctcccc gcacctccca cattctaccc ccgcccccgc ggacgttttg 1260
ataagtgatt gctttttaat gaccttgtca cctgagctag gaaggcctgg ggatctgggt 1320
ggaccccaaa gtcaggggct ttgactggga gggtgggggg ttctggaccc agggctggat 1380
gagctgttct gaaagaaagc atttgctctg gagtcccctg cctgtctcca ccctctgcag 1440
aaaatctcct gggcacattt tttcctgtca ggtgaagtca gccagcctag gaccttgtaa 1500
ccctgaacct tgtctccttt aacctccacc tctccaagtc ttgtctcact cccccaggct 1560
tagccaccct gtgctgtttt gggggctaaa aatagcacca cttcctcacc agctcaaaga 1620
atttgtcttc tttctttctc tcctgacctc ctgggcttgc tggccctgag gaagctggct 1680
gttgagacct catatcctgc tgtggttgtg gaaggcttcc ctgccctacg tgcccctctc 1740
agggggaaat agggtcaaca aacatagagt atggctcctg gtgggtcctg cctccttcag 1800
ggctggaggg gagatcctgg ttaggaaaga gaggaaagct agcaaattgc catgcgctga 1860
ggggattccg cagttccagc tctttcatgg gggatggttg ggccctgaac catgaagaaa 1920
aggtgacata ggggtcactg cttctggtca ccgtggtcca ctgcttacat ctgtcctgag 1980
gggataaggc gctgcttgcc agtgcttctc tctgaaccac atagcattgc ttaccgtctt 2040
ctaccagggc gtgtgtgtgt gtgtgcgtgt gtgtgtgtgt gtgtgtgttt taatgaaggc 2100
cctggatggg tgggagcacc ggaggcagga tatttttagg gggatgattt ctgatcagaa 2160
tctaggcctg cttcctctgc tcctatgaag gaagggggct atcccatgcc ttcctctgga 2220
gaatgtcttt tttgggggga cacccaaacc ccctttattc tacatccgcc tcctacgcaa 2280
tgcggtgttt gtggactcct tcttccttga ctcctggagg aggagcagaa aggattaagg 2340
gggattagga agggaggccc tggagggatg ggatgcaact gtaggcctta gaacctcatg 2400
ttggaaatgt atgcatttga cagtcatttc cattctcacc cccatctgtg gaaagggggc 2460
ctatctatga cattctgaag caaggtggca gtggacagtc atcacctctt tcttagcttt 2520
ccatctttcc tccactttac ttgggttcca ggtccccctt ttctggatct tcccttgttc 2580

ttctggaggt agttaatgtg ggcccagcta ctggaattag ttgaaacata gataggggtg 2640
tcttgggact ctgtttgcaa ggatgctggg caacagcatc taagtatgac ccccctgttg 2700
tcccttgctg cttcctgttt ctttcccact ttcaacatca taggaaggaa gaaaactaac 2760
atagagtatc tactgtgaga ttacattctc acatcaactc caagaaacag tctcccaatt 2820
taattatccc cagttaaaat agatggacac tgaggcttag ggaggttaac taccctgact 2880
gagatcagtt agtaagtggc agagttggga tttcaactta ggtcacccct aacagtcatg 2940
tgctttgccc tgaaccagga ttctccttgc cagtgtccct tcctgctgct caccctaaag 3000
agcatccaag atagctttca tctggggacc ttatgaggta gtatagggca tttcactata 3060
ttactgtcac cacaaaatta ataattaata tttattcatc atttttatgt gccaggtgac 3120
ttatggtaag gagcatagat acattatatc attcgaccct tactttcccc tttttaaaca 3180
agaagaaact gaggcttggt cccttagcta gtatttgcca aaacaggaag tcaaccccat 3240
gtctttgtga ctctggaagc tgagcattta cccattagac tatgttgaga gaaaatccct 3300
ccttgttgaa gcctgtaaac atgggacctg agggaaggac agtggaagcc cctgccttca 3360
gatgccatca gaggagggaa ggccacaggt tggagggctt gtcctcttac acatcctcct 3420
caactccacc cctagtaaga aacccaggca agactaaaga agggaattct ggtttctcat 3480
atttcctgtt tagttctttg cagattggtg ataaagcaag ggctggggtg ggtttggggg 3540
taggtgactg cgactttgga gcaaattcca caggccaggc ctccaaggtc aaagtaaggg 3600
gacttctatg tgagtgaccc ggttagccca gatagccatc cctttttagt gtgtggatgg 3660
gagcctaaag cgagaagacc atctctgcac cacatggtcc agatggggct ctgggttgag 3720
aggctttata ctggccttct tcaccacctt aggactcaca acttcctttt gctgtctgtt 3780
atttttttcta ggtctccaga ggcttgtgga agagaagcag gcgacccttc ctgagttatc 3840
ctggcttagc ctcccaatct ggctcccctt ccccttccca ttcccctgct ccccctgtcc 3900
cttccccatc cacccaactg aactgggtat aggtcaaagc tcctctcttt cctttccctt 3960
cctaggcact cattggctag gacctgtttg ctcttttttt tgtgcccaga gatactggaa 4020
cacgcttcat ctaagtaact gtggggaggg gtctttttga ctctacaagt ccttgagcaa 4080
aaagctgaaa aagaagcagg aggtggagaa gacccagtga agtgccccaa gccccatcat 4140
ggaagagggc ttccgagacc gggcagcttt catccgtggg gccaaagaca ttgctaagga 4200
agtcaaaaag catgcggcca agaaggtggt gaagggcctg gacagagtcc aggacgaata 4260
ttcccgaaga tcgtactccc gctttgagga ggaggatgat gatgatgact tccctgctcc 4320
cagtgatggt tattaccgag gagaagggac ccaggatgag gaggaaggtg gtgcatccag 4380
tgatgctact gagggccatg acgaggatga tgagatctat gaaggggaat atcagggcat 4440
tccccgggca gagtctgggg gcaaaggcga gcggatggca gatggggcgc ccctggctgg 4500
agtaaggggg ggcttgagtg atggggaggg tccccctggg ggccgggggg aggcacaacg 4560
acggaaagaa cgagaagaac tggcccaaca gtatgaagcc atcctacggg agtgtggcca 4620

cggccgcttc cagtggacac tgtattttgt gcttggtctg gcgctgatgg ctgacggtgt 4680
ggaggtcttt gtggtgggct tcgtgctgcc cagcgctgag aaagacatgt gcctgtccga 4740
ctccaacaaa ggcatgctag gtaagacatg gggggctcca gccttatccc ctcaaactct 4800
ggaaacctcc ttgctcctgg aaattgctct tgtccttacc actgggttcc ctaggaccct 4860
gcacccaaga tgttcccaga gggctcaggg ttcctctgca cactcttccc tcacacactc 4920
cctcttcttg gccttgggtc agggtgaagt tgaatgtgac agaggggctt gcgggagggc 4980
agctggactg gggcttgggc cagtggttgg gtggtggtct gcttcattct gcaggaggtg 5040
gctgtggtag gatggtccct accatttaat gtttcttcat agctttccct ctggttatcc 5100
tgttcccttt cactgattgg ggaacaagaa gccagtatcc aaactccttg gtataaatgg 5160
ggagagtcag tttggggtgg ttgggggagg gggggagggt cctggatctt ctgccagaga 5220
agctggttag actttctctc actcagatgg gagtttccag gggacaggat gtagtctttg 5280
atggcctttc ctgacaggga ctctgaggag gaaccccgta gactcacaga gaaagaatcg 5340
aggccttact ctgccaccaa attgccatgg ggtcttggac aggtctcttc ttcctggggc 5400
ttggtttggg catgggaaaa tgcaaagttt ggagtagatg attttaaaga ttaattccag 5460
ctttggtttt atgaaggcag tgaaaaacag gagagttttt ggaccagaaa tattgagact 5520
ggaaggagta aaggatggat tttgtgtggg agatttcctg ggaagatgaa atgaattgga 5580
gcccctttcc ccaggaacaa atgagagctc tcctgctgct ttgagggatc aaagcaggga 5640
gaatagagag gaccctgggg tggtctgtga tcctaggcac acccattcct ccttcaaagt 5700
gagccagggg taaaggtgtc actgtgtgtg tgtcagagtg agaaatgaga cagaccgaag 5760
agagtggaga gtttgcaggt tctcccatat cttcattcct gcctttgaac ccctggcctg 5820
cagagatgat cttctatctt tgcttacacc attcattttc tctcacttgc tctaatctct 5880
gggtagactt accttctaga agtttttcag atccttcagc tccctcagtg ctcatctcct 5940
gggagaatta aggtgtgtct gctcacaccc cctctgcctg ttttcctcct ccttccaggc 6000
ctcatcgtct acctgggcat gatggtggga gccttcctct ggggaggtct ggctgaccgg 6060
ctgggtcgga ggcagtgtct gctcatctcg ctctcagtca acagcgtctt cgccttcttc 6120
tcatcttttg tccagggtta cggcactttc ctcttctgcc gcctactttc tggggttggg 6180
tgagtgtaca cttcctaagt cccttgaggg caggactgtg ccttggtcac tgttgtatct 6240
ttagggccca gcacagtgcc aggcacacca aatgcacctt aaatctgtgt gagttgaacg 6300
gatgaatgag ttcctttctc ccccagccat cttcaggcca gctcctccct gcccaggacc 6360
tgtctgctgc tgtccagaat gctgttcctt ggcccactcc tgagtcctcc accccacctc 6420
tagttccatt tttgagcctg cctccacaaa gaggcctagt gggcctcagc cccacccctg 6480
ggctcctgtg gacccagagt taccccctac cagagtctac acatttcctg cattacacaa 6540
caaaccttgc taacacccac acctgccaga accctgacgc cttgtccgca ctgcccccttc 6600
acttttgcaa gcatggcagg cccgcttact ctgcttcaac tccaagctcc tttctggcca 6660

ttcccacaac atctgccctt ggaggctttt ggcttgtctt ttttctctgg agatttattc 6720
tcttttctgc aattactttc ccgcccttcc aactctgtaa ttcattttca agatttaaga 6780
tttgctcttt ccctttccct gagactcgct tgcctgtttc ccatggtccc aaggatgggt 6840
gcttgatggc tggcccacat gggagcttgt aaggctttat gtgtgagatg gagtctcttc 6900
aaaagcctgg aagacaaaag gggctcgggg tccagagcag tggggcaggg gatcaggact 6960
atggccattt gctaatgctg aattcttgaa atgcacttca ggattggagg gtccatcccc 7020
attgtcttct cctatttctc cgagtttctg gcccaggaga aacgagggga gcatttgagc 7080
tggctctgca tgttttggat gattggtggc gtgtacgcag ctgctatggc ctgggccatc 7140
atcccccact atggtgagca gcccccggaa atccacccca ggttatctcc ctcctccctc 7200
ttcttctgtg ctccctgtgg tgggttcccc cagcctctgg cctctctgct gatgctgtca 7260
ctttctccct accagggtgg agttttcaga tgggttctgc ctaccagttc cacagctgga 7320
gggtcttcgt cctcgtctgc gcctttcctt ctgtgtttgc cattggggct ctgaccacgc 7380
agcctgagag cccccgtttc ttcctagagg tgatggggct gagctgcatg ggtggggtg 7440
gggtgcgggc tgtggagtgt ggacggcagc aggggcttct gtgttcaaac accaactgga 7500
gtttgatttg atcctgtttg actgcctgcc ttcctcacag agtccactct ccaggggact 7560
ttatcttgca ttctttgcct tggaatgtgt ccctgagggg ttcttgggtg gagaaaccag 7620
gggacctctt cacccatgga tgacttaaga ttttgcaggg aagggccttg gccctgtcct 7680
gcatttacag ctcaagagac attcccaccc cctatacctt cccctcctaa ccccagcagg 7740
attaggtctt tgactacatg cttgcccatc atttccctta cctttggttt ttacattcct 7800
ctgtactatg gaattcccaa gacctgtgct tgcctttttc ccctggacaa cactccccac 7860
ccccaacccg cagctcttgc ccaagtctga ttgtcttgct atacctcagc ttggggctct 7920
ttcagagtgg tgagtgcatg cccataggtc agggcttggt ggtggggttg agggctggga 7980
accatttagt gtcctgtgac tacagaagcc aggtgggagg gagggggtct ataactctgg 8040
gtcaggtagg ggctgacttg gatgtgggga ttgtgtcttt ggctctagaa tggaaagcat 8100
gatgaggcct ggatggtgct gaagcaggtc catgatacca acatgcgagc caaaggacat 8160
cctgagcgag tgttctcagt aagccacagc cctcccgact cagacctcca cgccctgcag 8220
ccccagcccc tacctggccc ctctctgagt accccccacc tcagaccaca ctctaccgta 8280
aggagatttt cagggtatca tatccacctc ctgcctccca ttttgctaat gcaggggaaa 8340
ctgaggcgcc tggccgatgt tgggaaacac agagtgtgtg actgcccacc ccttctcttg 8400
accccaggta acccacatta agacgattca tcaggaggat gaattgattg agatccagtc 8460
ggacacaggg acctggtacc agcgctgggg ggtccgggcc ttgagcctag gggggcaggt 8520
aatggtggac ggggtggtag atagaaggga gtgggggaag tggagaagaa ggcaggactg 8580
aagtctatgt gtgtgggtga tgggtgagct gaagggaggt gggagggcag aaaagtgggg 8640
atctatgggt gctagaactt agtatagtca aggtctgact ttgtctggtc ccctgtaggt 8700

ttggggaat tttctctcct gttttggtcc cgaatatcgg cgcatcactc tgatgatgat 8760
gggtgtgtgg ttcaccatgt cattcaggtg aggagatggg tgggctgggt gatgtgtggt 8820
ggttgtggtg gtgtgtgggc tgggagagag gtggggagaa tggagatgca ggggacaagc 8880
gatgagggaa ggtgccatgc agaccccctc tgatcctgca gccagctcct ctacttcgct 8940
agttcactca acaaacttgt actgtaggct gaccaggatt cagtttctat tctgtgctgg 9000
gatgaaacag atgtggtcca aggtcctcct ggagtttatg ttttggttaa gaagcaggac 9060
gatgaggctc gtaatcccag cactttggga ggacgaggcg ggcggatcat gaggttagga 9120
gatcgagacc atcctggcta cggtgaaacc ccgtctctac taacatacaa aaaattagcc 9180
gggcatggtg gtgggcgcct gtagtcccag ctactctgga ggctgaggca ggagaatggt 9240
gtgaatccgg gaggcagagg ttgcagtgag tcgagatcgc gccactgcac tccagcctgg 9300
gtgacagagc gagactccgt ctcaaaagaa aaaaaagaag tgagacgaaa cttgctatga 9360
agcagggtga tgagagaagg gtgggggaag ggcagtggga gcacttgggg cttgggctgg 9420
ggtggggacc taagtcctca aaggtgaatg ggagtcagac aggtggagtg ggggtggctg 9480
gggtggagaa gagtgttttt ggcagaggga aaagcaaatg cttgggaggt aagaaaaagt 9540
atagtggctg ctgtgcgaga ccctgaagtc ccccaggagc gcagcaactg ttgaagggag 9600
gagcagggag aagctggtga ggctggagag gagcaaggtc ctaacagagg ttgactccat 9660
ttctcccttg ttctgagatg atacacattt ccgttttctc ctctgcctga ttctaatctc 9720
tgagccttgt ctgaagccct gttgtctgtt tccattgtcc ttgactcttc agctactatg 9780
gcctgaccgt ctggtttcct gacatgatcc gccatctcca ggcagtggac tacgcatccc 9840
gcaccaaagt gttccccggg gagcgcgtag agcatgtaac ttttaacttc acgttggaga 9900
atcagatcca ccgaggcggg cagtacttca atgacaagta tgtggggact tttaaacttc 9960
acttcttccc tgttccttct acctcgtctc ttttatgagt ctaggggttc aagacaagac 10020
atttgggggg caaacacttg tggtttcttc tcaatgccca gcttcccatt acttcgctaa 10080
agagtgagtc ttggaggggg catgaggatg acgggggagt gtctttcacc atcctggcct 10140
tacaggttca ttgggctgcg gctcaagtca gtgtcctttg aggattccct gtttgaagag 10200
tgttattttg aggatgtcac atccagcaac acgtttttcc gcaactgcac attcatcaac 10260
actgtgttct ataacactgg taaggcgggg tggctggtgg gtgtcagacc aaagggttgg 10320
gtggaccttg atgagaagga acctttttca ttcactatct tagggttctc aagctgagat 10380
ccctggaccc ctgtgggaag gtggcagagg ggcctgatag ctaccaccaa taattctgaa 10440
aatctaatgg atgttggaca ttttcctgta atagaatatc tgacaaaaac atcaagttta 10500
tttgcctttg gctgaaatta taactcagtg aggtaaaatt aatgcacttt tgaagaaggt 10560
aaaaatcatc caaaatagca ttcttgggaa aaagaaaaac aatcaataaa aggggtattt 10620
ggtgcggatc attgcatgaa tccactcctt tttaatagtg attctcaaac ttcagtatat 10680
gcacacgaaa tgctggtttt gtgagctaga ctgaaatttc ccacctcata ctgggcgagc 10740

tttttccatg ttgcaggaaa acaacaatgg ataggacttt ttattatttt aaatgttttt 10800
ctcagaaaaa tttcctaagt ccacggcatc gctactgctt atagtcagct agcacggaca 10860
gaaagaatga ataagcagct aagactatta atgggagaac ttggacaaag tcacactgtt 10920
ggacagatga tggtcatttt ctgctctaaa gatccctagt tcccatggtg atgttatttg 10980
atgttttgtg attagcactg tgaataattt tgagagcccc tcctactaac tttcatacat 11040
gcagagccct gagccataag ccgtaacagc ctccctctcc catctgctat gactgcctgc 11100
ttatcttggc ctttgcccac agacctgttc gagtacaagt ttgtgaacag ccgtctgata 11160
aacagtacat tcctgcacaa caaggagggc tgcccgctag acgtgacagg gacgggcgaa 11220
ggtgcctaca tggtatactt tgtgagcttc ctggggacac tggcagtgct tcctgggaat 11280
atcgtgtctg ccctgctcat ggacaagatc ggcaggctca gaatgcttgg taagggggag 11340
aggctgagtc agatctgata gtaggggcca ggggctgcgg gaggcagtca acaggcttct 11400
caccttgggc tgtggaagag attggtcaga gagttcctct ctcatgtttt ctgtttaggg 11460
ggtgctgcag gatggggtgg ggactggcac agaagcttct ctgggggtaa aggaggtaag 11520
cagaattgca agtggttagg gcacaggctt tgaggttatg ggttttgaat ctcagctcca 11580
ccacctgcct ctggcctctg gcaaattacc tctatgagcc tggttgtttc ctctgcaaaa 11640
tggaggtgat aatagtacct gtgtcatagg gttgatgtgc tagatcaatg catgtaaagc 11700
atcaattgcc ttggcccatg gctggcattg aataaatgat ggttactgat attattaata 11760
aacaattata agggaggttg aggaggagtg aaaagccttc cttttggata ccttggttgg 11820
ataccttcgg aacaagtctc atcctgggct atctgaaagc cagctgatgg gacagggtgg 11880
gctgtgggtg ggtttcactg tggccaggct catgttgctc tctcctctcc tggctccagc 11940
tggctccagc gtgatgtcct gtgtctcctg cttcttcctg tcttttggga acagtgagtc 12000
ggccatgatc gctctgctct gcctttttgg cggggtcagc attgcatcct ggaatgcgct 12060
ggacgtgttg actgttgaac tctacccctc agacaagagg tagttggaat gtgttggagg 12120
cagggcagtg gattcagggg aggggtgggg tgaggaagga gggctgaatg ggaagagcat 12180
ccttaggggt gagaggatat acatttccat tttctcctct aaaatctggt agctttggcc 12240
gggcacggtg actcatgcct gtaatcccag tgctttggga ggccgaggca ggcagatcac 12300
ctgaggtcag gagtttgaga ccagcctggc caacatggtg aaaccctgtc tgtactaaaa 12360
atacaaaaag ttagctgggt gcctgtaatc gcagctactc aggaggctaa ggcaggagaa 12420
ttgcttgaac ccgggaggtg gagctttcag tgagccgaga tcgcaccact gcactccagc 12480
ctgggcaaca gagcaagact ccgtctcaaa aaaaaaaaaa aaggaaaaaa aaatctggta 12540
gcttttgtat ctactcctca aagtcttaac tgttaacctc aatttttggt ctcctttccc 12600
tttgtctctc aatttattta cattcctggt gtacatcctt tacctctctg atctctccaa 12660
ccttctctct ccctcttgga cactgcctgc atcttcctgg acttcacttc cctgccctcc 12720
actcttcctt tgcctccctc ctgtaccttg cgccgtgctg cactggggga ctcctgagca 12780

ggaccacagc ttttggcttc ctgaatgccc tgtgtaagct ggcagctgtg ctggggatca 12840
gcatcttcac atccttcgtg ggaatcacca aggctgcacc catcctcttt gcctcagctg 12900
cccttgccct tggcagctct ctggccctga agctgcctga gacccggggg caggtgctgc 12960
agtgaagggg tctctagggc tttgggattg gcaggcacac tgtgagacca acaactcctt 13020
ccttcccctc cctgccctgc catcctgacc tccagagccc tcactcccca ctccccgtgt 13080
ttggtgtctt agctgtgtgt gcgtgtgcgt gtgcatgtgt gtaaaccccg tgggcaggga 13140
ctacagggaa ggctccttca tcccagtttt gagatgaagc tgtactcccc atttcccact 13200
gcccttgact ttgcacaaga gaaggctgag ccccatcctt ctccccctgt tagagagggg 13260
cccttgcttc cctgttccag gggttccaga ataggcttcc tgccttcccc atcattccct 13320
ctgcctaggc cctggtgaaa ccacaggtat gcaattatgc taggggctgg ggctctggtg 13380
tagaccatgg accaaaagaa cttcttagag tctgaagagt gggcctcggg tgccctctca 13440
catctcctgt tggatgctgg gggagaagca ataaacctca gccctctggc ctccactttc 13500
ctctcaattt gggctgcaaa tatgaagcct gaattttatg aaattagctt tctgattctt 13560
atttattaat agattaagtt ctgaggcagc tccgcaggac tgtgtgtgaa tgtgtatgta 13620
tacttacata tgtgtgtgca tgtgccatgg ggcggggggt atcactatac tgtcctcaaa 13680
tataagccaa gggtaatttc agcggatgca cacacaaccc tgcctcccac agttcctccc 13740
ctaatctggt ttctgtgttg agcctgggat ggaggagccc taggccagcc tgggataaga 13800
gtcccacagt ctagggagat ctgagggcat ccgacaaggc ccatctcctt ccctcctcaa 13860
gaagcagagg cctcctctgg agtgagaggc tccacccact acagcacagg cgggaatagc 13920
acagctgccc tcccatgctc cctacctgtc ccctcacagg gaggggagca ggggagggaa 13980
agaaaccagg catctggtca aaccagcaga tcaaaaagca caaagagctg gggcagaggc 14040
aggaagcagg ggccctcctg gcagctcctc tgagtgggga gaggttgggc agtgagtgag 14100
ggacccctaa tgcagggact agaagcctca gtttccccat tttacccttc cacacaatag 14160
cctctgtagg ttaggctgcc ccatcccacc ctactctgtg tggctgcttt ctttggtgcc 14220
ctcccctcac cccactgtag ctgtgacgtg ttgtagtttt tagatgtttg taaaatgttt 14280
aaaaaaatgt taaaaggaaa aaagtgaaaa taacaaaaaa gaaaatcaaa attcaccttc 14340
gtcatgctgc gtccagtgcc ccaaccctgt ggtcactctc cccattttgt aacact 14396

<210> 4
<211> 14
<212> DNA
<213> künstlich

<220>
<223> Oligonukleotid

<400> 4
aacatcggcc aggt 14

<210> 5
<211> 10
<212> DNA
<213> künstlich

<220>
<223> Oligonukleotid

<400> 5
gcctcagccc 10

SEQUENZPROTOKOLL

<110>  Desitin Arzneimittel GmbH

<120>  Schnelltest zum Nachweis von DNA-Sequenzen

<130>  P 72380

<160>  5

<170>  PatentIn version 3.3

<210>  1
<211>  1024
<212>  PRT
<213>  Escherichia coli

<220>
<223>  beta-Galactosidase

<400>  1
Met Thr Met Ile Thr Asp Ser Leu Ala Val Val Leu Gln Arg Arg Asp
1               5                   10                  15

Trp Glu Asn Pro Gly Val Thr Gln Leu Asn Arg Leu Ala Ala His Pro
            20                  25                  30

Pro Phe Ala Ser Trp Arg Asn Ser Glu Glu Ala Arg Thr Asp Arg Pro
        35                  40                  45

Ser Gln Gln Leu Arg Ser Leu Asn Gly Glu Trp Arg Phe Ala Trp Phe
    50                  55                  60

Pro Ala Pro Glu Ala Val Pro Glu Ser Trp Leu Glu Cys Asp Leu Pro
65                  70                  75                  80

Glu Ala Asp Thr Val Val Val Pro Ser Asn Trp Gln Met His Gly Tyr
                85                  90                  95

Asp Ala Pro Ile Tyr Thr Asn Val Thr Tyr Pro Ile Thr Val Asn Pro
            100                 105                 110

Pro Phe Val Pro Thr Glu Asn Pro Thr Gly Cys Tyr Ser Leu Thr Phe
        115                 120                 125

Asn Val Asp Glu Ser Trp Leu Gln Glu Gly Gln Thr Arg Ile Ile Phe
    130                 135                 140

Asp Gly Val Asn Ser Ala Phe His Leu Trp Cys Asn Gly Arg Trp Val
145                 150                 155                 160

Gly Tyr Gly Gln Asp Ser Arg Leu Pro Ser Glu Phe Asp Leu Ser Ala
                165                 170                 175

Phe Leu Arg Ala Gly Glu Asn Arg Leu Ala Val Met Val Leu Arg Trp
            180                 185                 190

Ser Asp Gly Ser Tyr Leu Glu Asp Gln Asp Met Trp Arg Met Ser Gly
        195                 200                 205

Ile Phe Arg Asp Val Ser Leu Leu His Lys Pro Thr Thr Gln Ile Ser
    210                 215                 220

Asp Phe His Val Ala Thr Arg Phe Asn Asp Asp Phe Ser Arg Ala Val
225                 230                 235                 240

Leu Glu Ala Glu Val Gln Met Cys Gly Glu Leu Arg Asp Tyr Leu Arg
                245                 250                 255

Val Thr Val Ser Leu Trp Gln Gly Glu Thr Gln Val Ala Ser Gly Thr
            260                 265                 270

Ala Pro Phe Gly Gly Glu Ile Ile Asp Glu Arg Gly Gly Tyr Ala Asp
        275                 280                 285

Arg Val Thr Leu Arg Leu Asn Val Glu Asn Pro Lys Leu Trp Ser Ala
    290                 295                 300

Glu Ile Pro Asn Leu Tyr Arg Ala Val Val Glu Leu His Thr Ala Asp
305                 310                 315                 320

Gly Thr Leu Ile Glu Ala Glu Ala Cys Asp Val Gly Phe Arg Glu Val
                325                 330                 335

Arg Ile Glu Asn Gly Leu Leu Leu Leu Asn Gly Lys Pro Leu Leu Ile
            340                 345                 350

Arg Gly Val Asn Arg His Glu His His Pro Leu His Gly Gln Val Met
        355                 360                 365

Asp Glu Gln Thr Met Val Gln Asp Ile Leu Leu Met Lys Gln Asn Asn
    370                 375                 380

Phe Asn Ala Val Arg Cys Ser His Tyr Pro Asn His Pro Leu Trp Tyr
385                 390                 395                 400

Thr Leu Cys Asp Arg Tyr Gly Leu Tyr Val Val Asp Glu Ala Asn Ile
                405                 410                 415

Glu Thr His Gly Met Val Pro Met Asn Arg Leu Thr Asp Asp Pro Arg
            420                 425                 430

Trp Leu Pro Ala Met Ser Glu Arg Val Thr Arg Met Val Gln Arg Asp
        435                 440                 445

Arg Asn His Pro Ser Val Ile Ile Trp Ser Leu Gly Asn Glu Ser Gly
    450                 455                 460

His Gly Ala Asn His Asp Ala Leu Tyr Arg Trp Ile Lys Ser Val Asp
465                 470                 475                 480

Pro Ser Arg Pro Val Gln Tyr Glu Gly Gly Gly Ala Asp Thr Thr Ala
                485                 490                 495

Thr Asp Ile Ile Cys Pro Met Tyr Ala Arg Val Asp Glu Asp Gln Pro
            500                 505                 510

Phe Pro Ala Val Pro Lys Trp Ser Ile Lys Lys Trp Leu Ser Leu Pro
        515                 520                 525

Gly Glu Thr Arg Pro Leu Ile Leu Cys Glu Tyr Ala His Ala Met Gly
    530                 535                 540

Asn Ser Leu Gly Gly Phe Ala Lys Tyr Trp Gln Ala Phe Arg Gln Tyr
545                 550                 555                 560

Pro Arg Leu Gln Gly Gly Phe Val Trp Asp Trp Val Asp Gln Ser Leu
                565                 570                 575

Ile Lys Tyr Asp Glu Asn Gly Asn Pro Trp Ser Ala Tyr Gly Gly Asp
            580                 585                 590

Phe Gly Asp Thr Pro Asn Asp Arg Gln Phe Cys Met Asn Gly Leu Val
        595                 600                 605

Phe Ala Asp Arg Thr Pro His Pro Ala Leu Thr Glu Ala Lys His Gln
    610                 615                 620

Gln Gln Phe Phe Gln Phe Arg Leu Ser Gly Gln Thr Ile Glu Val Thr
625                 630                 635                 640

Ser Glu Tyr Leu Phe Arg His Ser Asp Asn Glu Leu Leu His Trp Met
                645                 650                 655

Val Ala Leu Asp Gly Lys Pro Leu Ala Ser Gly Glu Val Pro Leu Asp
            660                 665                 670

Val Ala Pro Gln Gly Lys Gln Leu Ile Glu Leu Pro Glu Leu Pro Gln
        675                 680                 685

Pro Glu Ser Ala Gly Gln Leu Trp Leu Thr Val Arg Val Val Gln Pro
    690                 695                 700

Asn Ala Thr Ala Trp Ser Glu Ala Gly His Ile Ser Ala Trp Gln Gln
705                 710                 715                 720

Trp Arg Leu Ala Glu Asn Leu Ser Val Thr Leu Pro Ala Ala Ser His
                725                 730                 735

Ala Ile Pro His Leu Thr Thr Ser Glu Met Asp Phe Cys Ile Glu Leu
            740                 745                 750

Gly Asn Lys Arg Trp Gln Phe Asn Arg Gln Ser Gly Phe Leu Ser Gln
        755                 760                 765

Met Trp Ile Gly Asp Lys Lys Gln Leu Leu Thr Pro Leu Arg Asp Gln
    770                 775                 780

Phe Thr Arg Ala Pro Leu Asp Asn Asp Ile Gly Val Ser Glu Ala Thr
785                 790                 795                 800

Arg Ile Asp Pro Asn Ala Trp Val Glu Arg Trp Lys Ala Ala Gly His
                805                 810                 815

Tyr Gln Ala Glu Ala Ala Leu Leu Gln Cys Thr Ala Asp Thr Leu Ala
            820                 825                 830

Asp Ala Val Leu Ile Thr Thr Ala His Ala Trp Gln His Gln Gly Lys
        835                 840                 845

Thr Leu Phe Ile Ser Arg Lys Thr Tyr Arg Ile Asp Gly Ser Gly Gln
    850                 855                 860

Met Ala Ile Thr Val Asp Val Glu Val Ala Ser Asp Thr Pro His Pro
865                 870                 875                 880

Ala Arg Ile Gly Leu Asn Cys Gln Leu Ala Gln Val Ala Glu Arg Val
                885                 890                 895

Asn Trp Leu Gly Leu Gly Pro Gln Glu Asn Tyr Pro Asp Arg Leu Thr
            900                 905                 910

Ala Ala Cys Phe Asp Arg Trp Asp Leu Pro Leu Ser Asp Met Tyr Thr
        915                 920                 925

Pro Tyr Val Phe Pro Ser Glu Asn Gly Leu Arg Cys Gly Thr Arg Glu
    930                 935                 940

Leu Asn Tyr Gly Pro His Gln Trp Arg Gly Asp Phe Gln Phe Asn Ile
945                 950                 955                 960

Ser Arg Tyr Ser Gln Gln Gln Leu Met Glu Thr Ser His Arg His Leu
                965                 970                 975

Leu His Ala Glu Glu Gly Thr Trp Leu Asn Ile Asp Gly Phe His Met
            980                 985                 990

Gly Ile Gly Gly Asp Asp Ser Trp  Ser Pro Ser Val Ser Ala Glu Phe
        995                 1000                1005

Gln Leu  Ser Ala Gly Arg Tyr  His Tyr Gln Leu Val  Trp Cys Gln
    1010                1015                1020

Lys

<210> 2
<211> 2322
<212> DNA
<213> Staphylococcus aureus

<220>
<223> mecA-Gen

<400> 2
atgaaaaaga taaaaattgt tccacttatt ttaatagttg tagttgtcgg gtttggtata 60

tatttttatg cttcaaaaga taaagaaatt aataatacta ttgatgcaat tgaagataaa 120

aatttcaaac aagtttataa agatagcagt tatatttcta aaagcgataa tggtgaagta 180

gaaatgactg aacgtccgat aaaaatatat aatagtttag gcgttaaaga tataaacatt 240

caggatcgta aaataaaaaa agtatctaaa aataaaaaac gagtagatgc tcaatataaa 300

attaaaacaa actacggtaa cattgatcgc aacgttcaat ttaattttgt taaagaagat 360

ggtatgtgga agttagattg ggatcatagc gtcattattc caggaatgca gaaagaccaa 420

agcatacata ttgaaaattt aaaatcagaa cgtggtaaaa ttttagaccg aaacaatgtg 480

gaattggcca atacaggaac acatatgaga ttaggcatcg ttccaaagaa tgtatctaaa 540

aaagattata aagcaatcgc taaagaacta agtatttctg aagactatat caacaacaaa 600

tggatcaaaa ttgggtacaa gatgatacct tcgttccact ttaaaaccgt taaaaaaatg 660

gatgaatatt taagtgattt cgcaaaaaaa tttcatctta caactaatga aacagaaagt 720

cgtaactatc ctctaggaaa agcgacttca catctattag gttatgttgg tcccattaac 780

tctgaagaat taaaacaaaa agaatataaa ggctataaag atgatgcagt tattggtaaa 840

aagggactcg aaaaacttta cgataaaaag ctccaacatg aagatggcta tcgtgtcaca 900

atcgttagag tcgacgataa tagcaataca atcgcacata cattaataga gaaaaagaaa 960

aaagatggca aagatattca actaactatt gatgctaaag ttcaaaagag tatttataac 1020

aacatgaaaa atgattatgg ctcaggtact gctatccacc ctcaaacagg tgaattatta 1080

gcacttgtaa gcacaccttc atatgacgtc tatccattta tgtatggcat gagtaacgaa 1140

gaatataata aattaaccga agataaaaaa gaacctctgc tcaacaagtt ccagattaca 1200

acttcaccag gttcaactca aaaaatatta acagcaatga ttgggttaaa taacaaaaca 1260

ttagacgata aaacaagtta taaaatcgat ggtaaaggtt ggcaaaaaga taaatcttgg 1320

ggtggttaca acgttacaag atatgaagtg gtaaatggta atatcgactt aaaacaagca 1380

atagaatcat cagataacat tttctttgct agagtagcac tcgaattagg cagtaagaaa 1440

tttgaaaaag gcatgaaaaa actaggtgtt ggtgaagata taccaagtga ttatccattt 1500

tataatgctc aaatttcaaa caaaaattta gataatgaaa tattattagc tgattcaggt 1560

tacggacaag gtgaaatact gattaaccca gtacagatcc tttcaatcta tagcgcatta 1620

gaaaataatg gcaatattaa cgcacctcac ttattaaaag acacgaaaaa caaagtttgg 1680

aagaaaaata ttatttccaa agaaaatatc aatctattaa atgatggtat gcaacaagtc 1740

gtaaataaaa cacataaaga agatatttat agatcttatg caaacttaat tggcaaatcc 1800

ggtactgcag aactcaaaat gaaacaagga gaaactggca gacaaattgg gtggtttata 1860

tcatatgata aagataatcc aaacatgatg atggctatta atgttaaaga tgtacaagat 1920

aaaggaatgg ctagctacaa tgccaaaatc tcaggtaaag tgtatgatga gctatatgag 1980

aacggtaata aaaaatacga tatagatgaa taacaaaagc agtgaagcat ccgtaacgat 2040

ggttgcttca ctgttttatt atgaattatt aataagtgct gttacttctc cttaaataca 2100

tttctcattt catgtatgtt gaaagtgaca ctgtaacgag tccattttct ttttttatgg 2160

atttcttatt tgtaatttca gcgataacgt acaatgtatt acctgggtat acaggtttaa 2220

taaattttaa cgttattcat gttgtgttcc tgctacaact tcttctccgt atttaccttc 2280

ttctacccat aatttaaatg atattgaaag tgtattgcat gc 2322

<210> 3
<211> 14396
<212> DNA
<213> Homo sapiens

<220>
<223> SV2A-Gen

<400> 3
ttctccccgt gccctgactc cctccccatc ctcttttctc ctctccccac caatggtctc 60

tcccgatgct cagtggcgcg gggtaggggg gggcggaggc ggggctggag tggtggaagg 120

ggggtggcag gtctgcattg ccgcttccct ggtgccggga gcagtcgccg ctgccgcctc 180

cgcccgcggc cgggaccccc gtcctcgccc gggactcctt acccggggaa cctagaccag 240

gtgagacccc tttctcagtc gctcacctcc tccccgcccc tggcagaagc atctttgcct 300

tgccttgcag ccattttatc ctccgatgct ttgcttatgg ggagggtgac gatcccctct 360

ctaggggtga ggggggttgg tgggttggtg gtggtttggg gatggacatt tatttcagtt 420

gtgctcttca ggacacacac acacacacgc atatgtataa tattggtgaa tggggaggag 480
gggtgttttt ctctacctta accaagtccc cactatcccc ttcggagatt aacgtggtgg 540
tggggttgtg aggtgggatg aggttccctc aaactcctat cccctcctca ccttgacgtc 600
cccccacccc caggatgaat gataaatgac cttggctggg gctggagaat ggatgctgcc 660
tcagtgtgca cgcctggact tgggtgggtg gggggcaggg gaagaagggc aggtgaagga 720
tggagggaag gggagtgaag aatggaaata ctggggagac agagatgtag agccgccagg 780
tgttgggggc atggatggcg ggggctggcg gcagttgctc cggggtgggg ggaagggatg 840
ttgagtgaag gtggacactt gtctagcagc aggaccaagg gtggaggagg tgctcagagc 900
agggatttag tcagttctgg gggcttttcc cttattccgg agctgaggca agatggggtg 960
tgggggggtc gctgcaaatg ctgatagggg cggtctctat gtctgtgcat gcggaggtct 1020
ctccctgcgt ccccctctct gcctctccgt attcctcagt gtgcattctc agctgacacc 1080
ctcattttta tccctcagaa ctgtgggtgc tcctatcttc ctaaccttgg taggatgtgg 1140
ggaggggagg cgcagtcttg atggtcctgg gtgggggctg aagttcgatg accgggctgt 1200
catggggaag gggtgctccc gcacctccca cattctaccc ccgcccccgc ggacgttttg 1260
ataagtgatt gctttttaat gaccttgtca cctgagctag gaaggcctgg ggatctgggt 1320
ggaccccaaa gtcaggggct ttgactggga gggtgggggg ttctggaccc agggctggat 1380
gagctgttct gaaagaaagc atttgctctg gagtcccctg cctgtctcca ccctctgcag 1440
aaaatctcct gggcacattt tttcctgtca ggtgaagtca gccagcctag gaccttgtaa 1500
ccctgaacct tgtctccttt aacctccacc tctccaagtc ttgtctcact cccccaggct 1560
tagccaccct gtgctgtttt gggggctaaa aatagcacca cttcctcacc agctcaaaga 1620
atttgtcttc tttctttctc tcctgacctc ctgggcttgc tggccctgag gaagctggct 1680
gttgagacct catatcctgc tgtggttgtg gaaggcttcc ctgccctacg tgcccctctc 1740
agggggaaat agggtcaaca aacatagagt atggctcctg gtgggtcctg cctccttcag 1800
ggctggaggg gagatcctgg ttaggaaaga gaggaaagct agcaaattgc catgcgctga 1860
ggggattccg cagttccagc tctttcatgg gggatggttg ggccctgaac catgaagaaa 1920
aggtgacata ggggtcactg cttctggtca ccgtggtcca ctgcttacat ctgtcctgag 1980
gggataaggc gctgcttgcc agtgcttctc tctgaaccac atagcattgc ttaccgtctt 2040
ctaccagggc gtgtgtgtgt gtgtgcgtgt gtgtgtgtgt gtgtgtgttt taatgaaggc 2100

cctggatggg tgggagcacc ggaggcagga tatttttagg gggatgattt ctgatcagaa 2160
tctaggcctg cttcctctgc tcctatgaag gaagggggct atcccatgcc ttcctctgga 2220
gaatgtcttt tttgggggga cacccaaacc ccctttattc tacatccgcc tcctacgcaa 2280
tgcggtgttt gtggactcct tcttccttga ctcctggagg aggagcagaa aggattaagg 2340
gggattagga agggaggccc tggagggatg ggatgcaact gtaggcctta gaacctcatg 2400
ttggaaatgt atgcatttga cagtcatttc cattctcacc cccatctgtg gaaagggggc 2460
ctatctatga cattctgaag caaggtggca gtggacagtc atcacctctt tcttagcttt 2520
ccatctttcc tccactttac ttgggttcca ggtccccctt ttctggatct tcccttgttc 2580
ttctggaggt agttaatgtg ggcccagcta ctggaattag ttgaaacata gataggggtg 2640
tcttgggact ctgtttgcaa ggatgctggg caacagcatc taagtatgac ccccctgttg 2700
tcccttgctg cttcctgttt ctttcccact ttcaacatca taggaaggaa gaaaactaac 2760
atagagtatc tactgtgaga ttacattctc acatcaactc caagaaacag tctcccaatt 2820
taattatccc cagttaaaat agatggacac tgaggcttag ggaggttaac taccctgact 2880
gagatcagtt agtaagtggc agagttggga tttcaactta ggtcacccct aacagtcatg 2940
tgctttgccc tgaaccagga ttctccttgc cagtgtccct tcctgctgct caccctaaag 3000
agcatccaag atagctttca tctggggacc ttatgaggta gtatagggca tttcactata 3060
ttactgtcac cacaaaatta ataattaata tttattcatc atttttatgt gccaggtgac 3120
ttatggtaag gagcatagat acattatatc attcgaccct tactttcccc tttttaaaca 3180
agaagaaact gaggcttggt cccttagcta gtatttgcca aaacaggaag tcaaccccat 3240
gtctttgtga ctctggaagc tgagcattta cccattagac tatgttgaga gaaaatccct 3300
ccttgttgaa gcctgtaaac atgggacctg agggaaggac agtggaagcc cctgccttca 3360
gatgccatca gaggagggaa ggccacaggt tggagggctt gtcctcttac acatcctcct 3420
caactccacc cctagtaaga aacccaggca agactaaaga agggaattct ggtttctcat 3480
atttcctgtt tagttctttg cagattggtg ataaagcaag ggctggggtg ggtttggggg 3540
taggtgactg cgactttgga gcaaattcca caggccaggc ctccaaggtc aaagtaaggg 3600
gacttctatg tgagtgaccc ggttagccca gatagccatc cctttttagt gtgtggatgg 3660
gagcctaaag cgagaagacc atctctgcac cacatggtcc agatggggct ctgggttgag 3720
aggctttata ctggccttct tcaccacctt aggactcaca acttcctttt gctgtctgtt 3780

attttttcta ggtctccaga ggcttgtgga agagaagcag gcgacccttc ctgagttatc 3840
ctggcttagc ctcccaatct ggctcccctt ccccttccca ttcccctgct ccccctgtcc 3900
cttccccatc cacccaactg aactgggtat aggtcaaagc tcctctcttt ccttttcctt 3960
cctaggcact cattggctag gacctgtttg ctcttttttt tgtgcccaga gatactggaa 4020
cacgcttcat ctaagtaact gtggggaggg gtctttttga ctctacaagt ccttgagcaa 4080
aaagctgaaa aagaagcagg aggtggagaa gacccagtga agtgccccaa gccccatcat 4140
ggaagagggc ttccgagacc gggcagcttt catccgtggg gccaaagaca ttgctaagga 4200
agtcaaaaag catgcggcca agaaggtggt gaagggcctg gacagagtcc aggacgaata 4260
ttcccgaaga tcgtactccc gctttgagga ggaggatgat gatgatgact tccctgctcc 4320
cagtgatggt tattaccgag gagaagggac ccaggatgag gaggaaggtg gtgcatccag 4380
tgatgctact gagggccatg acgaggatga tgagatctat gaaggggaat atcagggcat 4440
tccccgggca gagtctgggg gcaaaggcga gcggatggca gatggggcgc ccctggctgg 4500
agtaaggggg ggcttgagtg atggggaggg tccccctggg ggccgggggg aggcacaacg 4560
acggaaagaa cgagaagaac tggcccaaca gtatgaagcc atcctacggg agtgtggcca 4620
cggccgcttc cagtggacac tgtattttgt gcttggtctg gcgctgatgg ctgacggtgt 4680
ggaggtcttt gtggtgggct tcgtgctgcc cagcgctgag aaagacatgt gcctgtccga 4740
ctccaacaaa ggcatgctag gtaagacatg gggggctcca gccttatccc ctcaaactct 4800
ggaaacctcc ttgctcctgg aaattgctct tgtccttacc actgggttcc ctaggaccct 4860
gcacccaaga tgttcccaga gggctcaggg ttcctctgca cactcttccc tcacacactc 4920
cctcttcttg gccttgggtc agggtgaagt tgaatgtgac agaggggctt gcgggagggc 4980
agctggactg gggcttgggc cagtggttgg gtggtggtct gcttcattct gcaggaggtg 5040
gctgtggtag gatggtccct accatttaat gtttcttcat agctttccct ctggttatcc 5100
tgttcccttt cactgattgg ggaacaagaa gccagtatcc aaactccttg gtataaatgg 5160
ggagagtcag tttggggtgg ttgggggagg gggggagggt cctggatctt ctgccagaga 5220
agctggttag actttctctc actcagatgg gagtttccag gggacaggat gtagtctttg 5280
atggcctttc ctgacaggga ctctgaggag gaaccccgta gactcacaga gaaagaatcg 5340
aggccttact ctgccaccaa attgccatgg ggtcttggac aggtctcttc ttcctggggc 5400
ttggtttggg catgggaaaa tgcaaagttt ggagtagatg attttaaaga ttaattccag 5460

ctttggtttt atgaaggcag tgaaaaacag gagagttttt ggaccagaaa tattgagact 5520

ggaaggagta aaggatggat tttgtgtggg agatttcctg ggaagatgaa atgaattgga 5580

gcccctttcc ccaggaacaa atgagagctc tcctgctgct ttgagggatc aaagcaggga 5640

gaatagagag gaccctgggg tggtctgtga tcctaggcac acccattcct ccttcaaagt 5700

gagccagggg taaaggtgtc actgtgtgtg tgtcagagtg agaaatgaga cagaccgaag 5760

agagtggaga gtttgcaggt tctcccatat cttcattcct gcctttgaac ccctggcctg 5820

cagagatgat cttctatctt tgcttacacc attcattttc tctcacttgc tctaatctct 5880

gggtagactt accttctaga agtttttcag atccttcagc tccctcagtg ctcatctcct 5940

gggagaatta aggtgtgtct gctcacaccc cctctgcctg ttttcctcct ccttccaggc 6000

ctcatcgtct acctgggcat gatggtggga gccttcctct ggggaggtct ggctgaccgg 6060

ctgggtcgga ggcagtgtct gctcatctcg ctctcagtca acagcgtctt cgccttcttc 6120

tcatcttttg tccagggtta cggcactttc ctcttctgcc gcctactttc tggggttggg 6180

tgagtgtaca cttcctaagt cccttgaggg caggactgtg ccttggtcac tgttgtatct 6240

ttagggccca gcacagtgcc aggcacacca aatgcacctt aaatctgtgt gagttgaacg 6300

gatgaatgag ttcctttctc ccccagccat cttcaggcca gctcctccct gcccaggacc 6360

tgtctgctgc tgtccagaat gctgttcctt ggcccactcc tgagtcctcc accccacctc 6420

tagttccatt tttgagcctg cctccacaaa gaggcctagt gggcctcagc cccacccctg 6480

ggctcctgtg gacccagagt taccccctac cagagtctac acatttcctg cattacacaa 6540

caaaccttgc taacacccac acctgccaga accctgacgc cttgtccgca ctgccccttc 6600

acttttgcaa gcatggcagg cccgcttact ctgcttcaac tccaagctcc tttctggcca 6660

ttcccacaac atctgccctt ggaggctttt ggcttgtctt ttttctctgg agatttattc 6720

tcttttctgc aattactttc ccgcccttcc aactctgtaa ttcattttca agatttaaga 6780

tttgctcttt ccctttccct gagactcgct tgcctgtttc ccatggtccc aaggatgggt 6840

gcttgatggc tggcccacat gggagcttgt aaggctttat gtgtgagatg gagtctcttc 6900

aaaagcctgg aagacaaaag gggctcgggg tccagagcag tggggcaggg gatcaggact 6960

atggccattt gctaatgctg aattcttgaa atgcacttca ggattggagg gtccatcccc 7020

attgtcttct cctatttctc cgagtttctg gcccaggaga aacgagggga gcatttgagc 7080

tggctctgca tgttttggat gattggtggc gtgtacgcag ctgctatggc ctgggccatc 7140

atcccccact atggtgagca gcccccggaa atccacccca ggttatctcc ctcctccctc 7200

ttcttctgtg ctccctgtgg tgggttcccc cagcctctgg cctctctgct gatgctgtca 7260

ctttctccct accagggtgg agttttcaga tgggttctgc ctaccagttc cacagctgga 7320

gggtcttcgt cctcgtctgc gcctttcctt ctgtgtttgc cattggggct ctgaccacgc 7380

agcctgagag cccccgtttc ttcctagagg tgatggggct gagctgcatg gggtggggtg 7440

gggtgcgggc tgtggagtgt ggacggcagc aggggcttct gtgttcaaac accaactgga 7500

gtttgatttg atcctgtttg actgcctgcc ttcctcacag agtccactct ccaggggact 7560

ttatcttgca ttctttgcct tggaatgtgt ccctgagggg ttcttgggtg gagaaaccag 7620

gggacctctt cacccatgga tgacttaaga ttttgcaggg aagggccttg gccctgtcct 7680

gcatttacag ctcaagagac attcccaccc cctatacctt cccctcctaa ccccagcagg 7740

attaggtctt tgactacatg cttgcccatc atttccctta cctttggttt ttacattcct 7800

ctgtactatg gaattcccaa gacctgtgct tgcctttttc ccctggacaa cactccccac 7860

ccccaacccg cagctcttgc ccaagtctga ttgtcttgct atacctcagc ttggggctct 7920

ttcagagtgg tgagtgcatg cccataggtc agggcttggt ggtggggttg agggctggga 7980

accatttagt gtcctgtgac tacagaagcc aggtgggagg gagggggtct ataactctgg 8040

gtcaggtagg ggctgacttg gatgtgggga ttgtgtcttt ggctctagaa tggaaagcat 8100

gatgaggcct ggatggtgct gaagcaggtc catgatacca acatgcgagc caaaggacat 8160

cctgagcgag tgttctcagt aagccacagc cctcccgact cagacctcca cgccctgcag 8220

ccccagcccc tacctggccc ctctctgagt accccccacc tcagaccaca ctctaccgta 8280

aggagatttt caggtatcaa tatccacctc ctgcctccca ttttgctaat gcaggggaaa 8340

ctgaggcgcc tggccgatgt tgggaaacac agagtgtgtg actgcccacc ccttctcttg 8400

accccaggta acccacatta agacgattca tcaggaggat gaattgattg agatccagtc 8460

ggacacaggg acctggtacc agcgctgggg ggtccgggcc ttgagcctag gggggcaggt 8520

aatggtggac ggggtggtag atagaaggga gtgggggaag tggagaagaa ggcaggactg 8580

aagtctatgt gtgtgggtga tgggtgagct gaagggaggt gggagggcag aaaagtgggg 8640

atctatgggt gctagaactt agtatagtca aggtctgact ttgtctggtc ccctgtaggt 8700

ttgggggaat tttctctcct gttttggtcc cgaatatcgg cgcatcactc tgatgatgat 8760

gggtgtgtgg ttcaccatgt cattcaggtg aggagatggg tgggctgggt gatgtgtggt 8820

ggttgtggtg gtgtgtgggc tgggagagag gtggggagaa tggagatgca ggggacaagc 8880
gatgagggaa ggtgccatgc agaccccctc tgatcctgca gccagctcct ctacttcgct 8940
agttcactca acaaacttgt actgtaggct gaccaggatt cagtttctat tctgtgctgg 9000
gatgaaacag atgtggtcca aggtcctcct ggagtttatg ttttggttaa gaagcaggac 9060
gatgaggctc gtaatcccag cactttggga ggacgaggcg ggcggatcat gaggttagga 9120
gatcgagacc atcctggcta cggtgaaacc ccgtctctac taacatacaa aaaattagcc 9180
gggcatggtg gtgggcgcct gtagtcccag ctactctgga ggctgaggca ggagaatggt 9240
gtgaatccgg gaggcagagg ttgcagtgag tcgagatcgc gccactgcac tccagcctgg 9300
gtgacagagc gagactccgt ctcaaaagaa aaaaaagaag tgagacgaaa cttgctatga 9360
agcagggtga tgagagaagg gtgggggaag ggcagtggga gcacttgggg cttgggctgg 9420
ggtggggacc taagtcctca aaggtgaatg ggagtcagac aggtggagtg ggggtggctg 9480
gggtggagaa gagtgttttt ggcagaggga aaagcaaatg cttgggaggt aagaaaaagt 9540
atagtggctg ctgtgcgaga ccctgaagtc ccccaggagc gcagcaactg ttgaagggag 9600
gagcagggag aagctggtga ggctggagag gagcaaggtc ctaacagagg ttgactccat 9660
ttctcccttg ttctgagatg atacacattt ccgttttctc ctctgcctga ttctaatctc 9720
tgagccttgt ctgaagccct gttgtctgtt tccattgtcc ttgactcttc agctactatg 9780
gcctgaccgt ctggtttcct gacatgatcc gccatctcca ggcagtggac tacgcatccc 9840
gcaccaaagt gttccccggg gagcgcgtag agcatgtaac ttttaacttc acgttggaga 9900
atcagatcca ccgaggcggg cagtacttca atgacaagta tgtggggact tttaaacttc 9960
acttcttccc tgttccttct acctcgtctc ttttatgagt ctaggggttc aagacaagac 10020
atttgggggg caaacacttg tggtttcttc tcaatgccca gcttcccatt acttcgctaa 10080
agagtgagtc ttggaggggg catgaggatg acgggggagt gtctttcacc atcctggcct 10140
tacaggttca ttgggctgcg gctcaagtca gtgtcctttg aggattccct gtttgaagag 10200
tgttattttg aggatgtcac atccagcaac acgtttttcc gcaactgcac attcatcaac 10260
actgtgttct ataacactgg taaggcgggg tggctggtgg gtgtcagacc aaagggttgg 10320
gtggaccttg atgagaagga acctttttca ttcactatct tagggttctc aagctgagat 10380
ccctggaccc ctgtgggaag gtggcagagg ggcctgatag ctaccaccaa taattctgaa 10440
aatctaatgg atgttggaca ttttcctgta atagaatatc tgacaaaaac atcaagttta 10500

tttgcctttg gctgaaatta taactcagtg aggtaaaatt aatgcacttt tgaagaaggt 10560

aaaaatcatc caaaatagca ttcttgggaa aaagaaaaac aatcaataaa aggggtattt 10620

ggtgcggatc attgcatgaa tccactcctt tttaatagtg attctcaaac ttcagtatat 10680

gcacacgaaa tgctggtttt gtgagctaga ctgaaatttc ccacctcata ctgggcgagc 10740

tttttccatg ttgcaggaaa acaacaatgg ataggacttt ttattatttt aaatgttttt 10800

ctcagaaaaa tttcctaagt ccacggcatc gctactgctt atagtcagct agcacggaca 10860

gaaagaatga ataagcagct aagactatta atgggagaac ttggacaaag tcacactgtt 10920

ggacagatga tggtcatttt ctgctctaaa gatccctagt tcccatggtg atgttatttg 10980

atgttttgtg attagcactg tgaataattt tgagagcccc tcctactaac tttcatacat 11040

gcagagccct gagccataag ccgtaacagc ctccctctcc catctgctat gactgcctgc 11100

ttatcttggc ctttgcccac agacctgttc gagtacaagt ttgtgaacag ccgtctgata 11160

aacagtacat tcctgcacaa caaggagggc tgcccgctag acgtgacagg gacgggcgaa 11220

ggtgcctaca tggtatactt tgtgagcttc ctggggacac tggcagtgct tcctgggaat 11280

atcgtgtctg ccctgctcat ggacaagatc ggcaggctca gaatgcttgg taagggggag 11340

aggctgagtc agatctgata gtaggggcca ggggctgcgg gaggcagtca acaggcttct 11400

caccttgggc tgtggaagag attggtcaga gagttcctct ctcatgtttt ctgtttaggg 11460

ggtgctgcag gatggggtgg ggactggcac agaagcttct ctgggggtaa aggaggtaag 11520

cagaattgca agtggttagg gcacaggctt tgaggttatg ggttttgaat ctcagctcca 11580

ccacctgcct ctggcctctg gcaaattacc tctatgagcc tggttgtttc ctctgcaaaa 11640

tggaggtgat aatagtacct gtgtcatagg gttgatgtgc tagatcaatg catgtaaagc 11700

atcaattgcc ttggcccatg gctggcattg aataaatgat ggttactgat attattaata 11760

aacaattata agggaggttg aggaggagtg aaaagccttc cttttggata ccttggttgg 11820

ataccttcgg aacaagtctc atcctgggct atctgaaagc cagctgatgg gacagggtgg 11880

gctgtgggtg ggtttcactg tggccaggct catgttgctc tctcctctcc tggctccagc 11940

tggctccagc gtgatgtcct gtgtctcctg cttcttcctg tcttttggga acagtgagtc 12000

ggccatgatc gctctgctct gcctttttgg cggggtcagc attgcatcct ggaatgcgct 12060

ggacgtgttg actgttgaac tctacccctc agacaagagg tagttggaat gtgttggagg 12120

cagggcagtg gattcagggg aggggtgggg tgaggaagga gggctgaatg ggaagagcat 12180

ccttaggggt gagaggatat acatttccat tttctcctct aaaatctggt agctttggcc 12240
gggcacggtg actcatgcct gtaatcccag tgctttggga ggccgaggca ggcagatcac 12300
ctgaggtcag gagtttgaga ccagcctggc caacatggtg aaaccctgtc tgtactaaaa 12360
atacaaaaag ttagctgggt gcctgtaatc gcagctactc aggaggctaa ggcaggagaa 12420
ttgcttgaac ccgggaggtg gagctttcag tgagccgaga tcgcaccact gcactccagc 12480
ctgggcaaca gagcaagact ccgtctcaaa aaaaaaaaaa aaggaaaaaa aaatctggta 12540
gcttttgtat ctactcctca aagtcttaac tgttaacctc aatttttggt ctcctttccc 12600
tttgtctctc aatttattta cattcctggt gtacatcctt tacctctctg atctctccaa 12660
ccttctctct ccctcttgga cactgcctgc atcttcctgg acttcacttc cctgccctcc 12720
actcttcctt tgcctccctc ctgtaccttg cgccgtgctg cactggggga ctcctgagca 12780
ggaccacagc ttttggcttc ctgaatgccc tgtgtaagct ggcagctgtg ctggggatca 12840
gcatcttcac atccttcgtg ggaatcacca aggctgcacc catcctcttt gcctcagctg 12900
cccttgccct tggcagctct ctggccctga agctgcctga gacccggggg caggtgctgc 12960
agtgaagggg tctctagggc tttgggattg gcaggcacac tgtgagacca acaactcctt 13020
ccttcccctc cctgccctgc catcctgacc tccagagccc tcactcccca ctccccgtgt 13080
ttggtgtctt agctgtgtgt gcgtgtgcgt gtgcatgtgt gtaaaccccg tgggcaggga 13140
ctacagggaa ggctccttca tcccagtttt gagatgaagc tgtactcccc atttcccact 13200
gcccttgact ttgcacaaga gaaggctgag ccccatcctt ctccccctgt tagagagggg 13260
cccttgcttc cctgttccag gggttccaga ataggcttcc tgccttcccc atcattccct 13320
ctgcctaggc cctggtgaaa ccacaggtat gcaattatgc taggggctgg ggctctggtg 13380
tagaccatgg accaaaagaa cttcttagag tctgaagagt gggcctcggg tgccctctca 13440
catctcctgt tggatgctgg gggagaagca ataaacctca gccctctggc ctccactttc 13500
ctctcaattt gggctgcaaa tatgaagcct gaattttatg aaattagctt tctgattctt 13560
atttattaat agattaagtt ctgaggcagc tccgcaggac tgtgtgtgaa tgtgtatgta 13620
tacttacata tgtgtgtgca tgtgccatgg ggcggggggt atcactatac tgtcctcaaa 13680
tataagccaa gggtaatttc agcggatgca cacacaaccc tgcctcccac agttcctccc 13740
ctaatctggt ttctgtgttg agcctgggat ggaggagccc taggccagcc tgggataaga 13800
gtcccacagt ctagggagat ctgagggcat ccgacaaggc ccatctcctt ccctcctcaa 13860

gaagcagagg cctcctctgg agtgagaggc tccacccact acagcacagg cgggaatagc 13920

acagctgccc tcccatgctc cctacctgtc ccctcacagg gaggggagca ggggagggaa 13980

agaaaccagg catctggtca aaccagcaga tcaaaaagca caaagagctg gggcagaggc 14040

aggaagcagg ggccctcctg gcagctcctc tgagtgggga gaggttgggc agtgagtgag 14100

ggacccctaa tgcagggact agaagcctca gtttccccat tttacccttc cacacaatag 14160

cctctgtagg ttaggctgcc ccatcccacc ctactctgtg tggctgcttt ctttggtgcc 14220

ctcccctcac cccactgtag ctgtgacgtg ttgtagtttt tagatgtttg taaaatgttt 14280

aaaaaaatgt taaaaggaaa aaagtgaaaa taacaaaaaa gaaaatcaaa attcaccttc 14340

gtcatgctgc gtccagtgcc ccaaccctgt ggtcactctc cccattttgt aacact 14396

<210> 4
<211> 15
<212> DNA
<213> künstlich

<220>
<223> Oligonukleotid

<400> 4
aacatcggcc aggt 15

<210> 5
<211> 10
<212> DNA
<213> künstlich

<220>
<223> Oligonukleotid

<400> 5
gcctcagccc 10

## Beschreibung

### GEBIET DER ERFINDUNG

Die vorliegende Erfindung betrifft in vitro-Verfahren zum Nachweis einer DNA-Zielsequenz in Zellen unter Verwendung eines Oligonukleotids, das mit einem beta-D-Galactopyranosid markiert ist, welches bei Hydrolyse der glycosidischen Bindung einen wasserunlöslichen Farbstoff bildet. Die Erfindung betrifft insbesondere ein in vitro-Verfahren zum Nachweis einer DNA-Zielsequenz aus einer Gruppe von DNA-Sequenzen, deren Mitglieder sich in genau einer vorbestimmten Nukleotid-Position voneinander unterscheiden, unter Verwendung eines Oligonukleotids, das mit einem beta-D-Galactopyranosid markiert ist, welches bei Hydrolyse der glycosidischen Bindung einen wasserunlöslichen Farbstoff bildet. Die Erfindung betrifft außerdem die Verwendung eines solchen Oligonukleotids in DNA-Hybridisierungsverfahren. Schließlich betrifft die Erfindung Kits zur Durchführung der obigen Verfahren.

### HINTERGRUND DER ERFINDUNG

Der Nachweis von bestimmten DNA-Sequenzen in biologischen Proben spielt auf dem Gebiet der Molekularbiologie eine entscheidende Rolle. So werden beispielsweise in der human- und veterinärmedizinischen Praxis zahlreiche Infektionen durch Nachweis von erregerspezifischen DNA-Sequenzen diagnostiziert. Ferner werden Verfahren zum Nachweis bestimmter DNA-Sequenzen auch bei der Identifizierung genetischer Varianten von bestimmten humanen Gene eingesetzt. Solche genetischen Varianten können als Marker für den Erkrankungsverlauf und für die Therapieresponse bei einer Reihe von Krankheiten eingesetzt werden, z.B. bei psychiatrischen und neurologischen Erkrankungen.

Üblicherweise werden zum Nachweis von DNA-Sequenzen in der Erregerdiagnostik Amplifikationsverfahren, wie beispielsweise PCR oder RT-PCR, eingesetzt, um die Menge der in der Probe vorliegenden erregerspezifischen DNA zu erhöhen. In einem ersten Schritt wird dabei üblicherweise ausgehend von der jeweiligen Patientenprobe (beispielsweise Blut, Serum, Gewebe, o.ä.) eine DNA-Aufreinigung durchgeführt. Die aufgereinigte DNA kann je nach Art des nachzuweisenden Erregers aus bakteriellen Zellen stammen oder aus Zellen des betroffenen Gewebes des jeweiligen Patienten (z.B. bei Infektion mit Viren, deren DNA sich in die genomische DNA der infizierten Wirtszellen integriert). Die aufgereinigte DNA kann anschließend in das jeweilige Amplifikationsverfahren eingesetzt werden, wobei Primer verwendet werden, die hinsichtlich ihrer Sequenz für den nachzuweisenden Erreger spezifisch sind.

Eine weitere Möglichkeit zum Nachweis von erregerspezifischen DNA-Sequenzen bieten DNA-Hybridisierungsverfahren, bei denen das Vorliegen einer definierten DNA-Sequenz durch Hybridisierung mit markierten Sonden nachgewiesen wird. Ein klassischer Weg zu Durchführung von DNA-Hybridisierungsverfahren ist das Southern-Blotting. Bei dieser Methode wird die aus den Zellen bzw. Geweben isolierte DNA nach Fragmentierung mit Restriktionsendonukleasen mittels Gelelektrophorese aufgetrennt, auf eine Membran (z.B. eine Nitrocellulose- oder Nylonmembran) übertragen, immobilisiert und mit einer entsprechend markierten Oligonukleotid-Sonde hybridisiert. Alternativ dazu kann die isolierte DNA direkt auf eine Membran gegeben und anschließend hybridisiert werden (Dot-Blot). Das Southern-Blotting wird häufig zur Untersuchung von Gen-Umlagerungen verwendet.

Neben dem Southern-Blotting hat sich in der Erregerdiagnostik auch die in-situ Hybridisierung etabliert. Unter in-situ Hybridisierung versteht man den direkten Nachweis von Nukleinsäuresequenzen in Schnitt- und Zellpräparaten unter Verwendung geeigneter Sonden. Dieses Verfahren wird in vielen Laboratorien bereits zum Nachweis viraler DNS (Cytomegalievirus, Herpesviren, etc.) in Gewebeschnitten und Zellen eingesetzt. Das Verfahren erlaubt darüber hinaus auch die Analyse von strukturellen chromosomalen Veränderungen.

Die bei DNA-Hybridisierungsverfahren verwendeten Sonden können auf verschiedene Arten markiert sein, wie beispielsweise durch fluoreszierende Moleküle oder durch Gruppen, die sich immunologisch über eine Reaktion mit einem Antikörper nachweisen lassen. Auch Hybridisierungsverfahren erfordern in der Regel die Aufreinigung der zu untersuchenden DNA.

Die bislang im Stand der Technik beschriebenen Verfahren zum DNA-Nachweis weisen den Nachteil auf, dass verhältnismäßig aufwendige Apparaturen, wie beispielsweise PCR-Thermocycler, Fluoreszenzmikroskop oder ähnliches erforderlich sind, um Aussagen bezüglich des Vorliegens der nachzuweisenden DNA-Sequenz treffen zu können. Darüber hinaus erlauben diese Verfahren eine Auswertung erst mit einer erheblichen zeitlichen Verzögerung, die sich aus den nachgeschalteten Verfahren ergibt. Die Verfahren erfordern ferner in der Regel zunächst eine Aufreinigung der verwendeten DNA, was mit einem weiteren Zeit- und Kostenaufwand verbunden ist.

Aufgabe der vorliegenden Erfindung ist es daher, Verfahren zur Verfügung zu stellen, mit denen man verläßlich und reproduzierbar das Vorhandensein bestimmter DNA-Sequenzen in Zellen nachweisen kann. Die Verfahren sollten ohne aufwendige apparative Ausstattung durchführbar sein, und das Ergebnis sollte möglichst innerhalb von wenigen Minuten verfügbar sein. Erfindungsgemäß wird die obige Aufgabe durch den Gegenstand der vorliegenden Patentansprüche gelöst.

Es wurde nunmehr überraschenderweise festgestellt, dass sich Oligonukleotide, die mit einem beta-D-Galactopyranosid markiert sind, welches bei Hydrolyse der glycosidischen Bindung einen in Wasser unlöslichen Farbstoff bildet, in vorteilhafter Weise in einem Hybridisierungsassay zum Nachweis von DNA-Sequenzen einsetzen lassen. In einem wässrigen Milieu läßt sich der gebildete Farbstoff mit bloßem Auge lediglich im Bereich von lipophilen Strukturen, wie z.B. lipidhaltigen Membranen von lysierten Zellen, erkennen, wohingegen er in der wässrigen Reaktionslösung zunächst nicht zu einer wahrnehmbaren Verfärbung führt. Somit eignen sich die erfindungsgemäß markierten Oligonukleotide insbesondere zur Verwendung in einem colorimetrischen Schnelltest, bei dem vollständig oder teilweise lysierte prokaryontische und/oder eukaryontische Zellen, die sich auf einem geeigneten Träger befinden, in eine Reaktionslösung eingebracht werden, die erfindungsgemäß markierte Oligonukleotide als Sonden enthält. Nach Hybridisierung der Oligonukleotide an die aus den Zellen freigesetzte DNA erfolgt die Spaltung des beta-D-Galactopyranosids und die Ausbildung des Farbstoffs im Bereich der lysierten Zellen. Die Spaltung kann beispielsweise enzymatisch durch Zugabe oder Freisetzung eines Polypeptids mit beta-Galactosidaseaktivität erfolgen.

Bei einem solchen Schnelltest ist es nicht erforderlich, vor dem Hybridisieren der markierten Oligonukleotide an die DNA-Zielsequenz eine Aufreinigung der DNA aus den jeweiligen Zellen durchzuführen. Die Zellen werden vielmehr direkt im (teilweise) lysierten Zustand in die Reaktionslösung eingesetzt. Überraschenderweise ist es ebenfalls nicht erforderlich, vor der Durchführung der Farbreaktion die an die DNA der Zellen hybridisierten Oligonukleotid-Sonden von ungebundenen Sonden abzutrennen, die in der Reaktionslösung vorliegen. Die frei in der Lösung vorliegenden Sondenmoleküle führen erst nach einer erheblichen zeitlichen Verzögerung zu einer Anfärbung der Reaktionslösung. Erfindungsgemäß wird somit ein stabiles und reproduzierbares Nachweissystem bereitgestellt, das innerhalb von Minuten Aufschluß über das Vorliegen der gesuchten DNA-Sequenz in den untersuchten Zellen gibt.

### BESCHREIBUNG DER FIGUREN

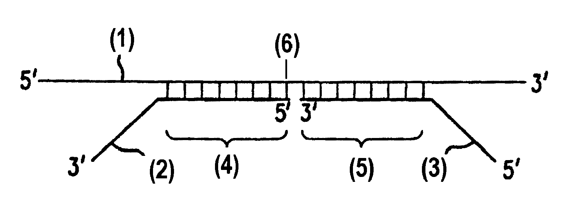

Figur 1 zeigt das Prinzip der Ligations-Detektionsreaktion (LDR). Eine DNA-Zielsequenz (1) wird mit zwei Oligonukleotiden (2) und (3) unter Hybridisierungsbedingungen inkubiert. Die beiden Oligonukleotide weisen mindestens einen endständigen Abschnitt (4) bzw. (5) auf, in dem sie ausreichend komplementär zu der DNA-Zielsequenz sind, um eine Hybridisierung der Oligonukleotide an die DNA-Zielsequenz zu ermöglichen. Nur wenn in dem Bereich des Duplexes, in dem die beiden Oligonukleotide unmittelbar aneinandergrenzen, eine vollständige Komplementarität zur DNA-Zielsequenz (1) besteht, ist eine Ligation der Oligonukleotide und eine nachfolgende PCR-Amplifikation der Gesamtsequenz, die sich aus den beiden Oligonukleotiden zusammensetzt, möglich.

### BESCHREIBUNG DER ERFINDUNG

Die Erfindung betrifft Verfahren zum Nachweis bestimmter DNA-Sequenzen unter Verwendung von Oligonukleotiden, die mit einem beta-D-Galactopyranosid markiert sind, welches bei Hydrolyse der glycosidischen Bindung einen in Wasser unlöslichen Farbstoff bildet. Die Erfindung stellt daher ein in vitro-Verfahren zum Nachweis einer DNA-Zielsequenz in Zellen bereit, bei dem man
(a) die Zellen auf einem Träger bereitstellt, wobei mindestens ein Teil der Zellen lysiert ist;
(b) die auf dem Träger bereitgestellten Zellen mit mindestens einem Oligonukleotid in Kontakt bringt, das in der Lage ist, mit der DNA-Zielsequenz zu hybridisieren, wobei das Oligonukleotid mit einem beta-D-Galactopyranosid markiert ist, das bei Hydrolyse der glycosidischen Bindung einen wasserunlöslichen Farbstoff bildet;
(c) das mindestens eine Oligonukleotid unter Bedingungen inkubiert, die eine Hybridisierung des mindestens einen Oligonukleotids an die DNA-Zielsequenz ermöglichen;
(d) das Hybridisierungsprodukt mittels Hydrolyse der glycosidischen Bindung des beta-D-Galactopyranosids nachweist,
wobei die Bildung eines Farbstoffs im Bereich der auf dem Träger befindlichen Zellen auf das Vorliegen der DNA-Zielsequenz in den Zellen hinweist.

Das erfindungsgemäße Verfahren richtet sich auf den Nachweis einer DNA-Zielsequenz, d.h. einer DNA-Sequenz von zumindest teilweise bekannter Nukleotid- bzw. Basenabfolge. Bei der DNA-Zielsequenz kann es sich beispielsweise um ein Gen oder um einen Teil eines Gens handeln. Die DNA-Zielsequenz, die durch die erfindungsgemäßen Verfahren nachgewiesen werden soll, hat vorzugsweise eine Länge von etwa 20-20000 Nukleotiden, wobei eine Länge von etwa 50, 60, 70, 80, 90, 100, 200, 300, 400, 500, 600, 1000, 5000 oder 10000 Nukleotiden besonders bevorzugt ist. Als Nukleotid wird vorliegend der Baustein der DNA verstanden, der aus einer Phosphorsäure, einer Pentose und einer der vier Basen Adenin, Cytosin, Guanin und Thymin besteht. Die DNA-Zielsequenz kann prokaryontischen, eukaryontischen oder viralen Ursprungs sein. Das Vorliegen, Fehlen, sowie die konkrete Zusammensetzung der für die Untersuchung ausgewählten DNA-Zielsequenz kann beispielsweise Rückschlüsse über das Vorliegen einer bestimmten Erkrankung (z.B. einer genetischen Erbkrankheit oder einer Infektionskrankheit) erlauben, oder es kann klinisch relevante Parameter liefern, die den weiteren Verlauf einer therapeutischen Behandlung beeinflussen können (z.B. das Vorliegen einer Resistenz gegen bestimmte Antibiotika). Es lassen sich anhand der erfindungsgemäßen Verfahren insbesondere DNA-Sequenzen bakteriellen und/oder viralen Ursprungs in biologischen Proben identifizieren, wie beispielsweise in Gewebeproben eines Säugetiers. Andererseits lassen sich durch Verwendung geeigneter Sonden auch Sequenzabweichung, wie beispielsweise Einzelnukleotid-Polymorphismen, in einer DNA-Sequenz nachweisen.

In einem ersten Schritt des erfindungsgemäßen Verfahrens werden die zu untersuchenden Zellen, von denen angenommen wird, dass sie die nachzuweisende DNA-Zielsequenz enthalten könnten (Zielzellen), auf einem Träger bereitgestellt, wobei mindestens ein Teil der Zellen lysiert ist. Dies bedeutet, dass die äußere Hülle der Zelle (sowie bei eukaryotischen Zellen die Hülle des Zellkerns) zerstört wurde, sodass die in der Zelle befindliche DNA freigesetzt wurde und somit für die weiteren Verfahrensschritte zugänglich ist. Vorzugsweise liegt die DNA dabei in einzelsträngiger Form vor. Die DNA bleibt für einen ausreichenden Zeitraum an dem Träger haften und geht nicht in Lösung. Je nach Art der nachzuweisenden DNA-Zielsequenz können die zu untersuchenden Zellen verschiedener Herkunft sein. In der Regel werden die zu untersuchenden Zielzellen in Form einer Mischprobe vorliegen, in der neben den Zielzellen auch andere Arten von Zellen vorliegen. Dies stört die Verfahren der vorliegenden Erfindung nicht, solange gewährleistet ist, dass eine genügend hohe Anzahl von Zielzellen in das Verfahren eingesetzt wird. Vorzugsweise handelt es sich bei den in das Verfahren der vorliegenden Erfindung eingesetzten Zellen um solche, die aus einem Abstrich, wie z.B. aus einem Abstrich der Mundschleimhaut, stammen.

Sofern es sich bei der nachzuweisenden DNA-Zielsequenz um eine DNA-Sequenz eines pathogenen Bakteriums handelt, kann es sich bei den Zellen, die auf den Träger aufgebracht werden, um solche handeln, die aus einer Probe von einem potentiell mit dem Bakterium infizierten Patienten, vorzugsweise aus einem humanen Patienten, erhalten worden sind. Sofern beispielsweise eine DNA-Zielsequenz aus Bakterien nachgewiesen werden soll, die üblicherweise den Nasen- und/oder Rachenraum besiedeln, können die Zellen in Form einer Mischprobe (d.h. gemeinsam mit eukaryontischen Zellen, wie beispielsweise Schleimhautzellen des Patienten) durch Entnahme einer Gewebeprobe aus diesem Körperbereich des Patienten oder mittels eines Abstrichs gewonnen werden. Die gesamte Mischprobe kann unmittelbar auf den Träger aufgebracht werden, wobei eine Trennung der verschiedenen in der Probe vorliegenden Zellen nicht notwendig ist.

Ein Beispiel für ein in der medizinischen Praxis relevantes pathogenes Bakterium, welches den Nasen- und Rachenraum des Menschen besiedelt, ist *Staphylococcus aureus.* Bestimmte Stämme des grampositiven Mikroorganismus weisen eine Resistenz gegen zahlreiche in der klinischen Therapie bedeutsame Antibiotika auf. Insbesondere die als Methicillin-resistente *Staphylococcus aureus* (MRSA) bezeichneten Stämme haben sich in den vergangenen Jahren zu einem ernstzunehmenden Problem entwikkelt. MRSA ist für eine Vielzahl von nosokomialen Infektionen verantwortlich, die eine Verlängerung der Liegedauer des Patienten bedingen und damit zu deutlich erhöhten Kosten für Krankenhäuser führen. Die Verbreitung erfolgt in der Regel durch Patienten oder Pflegepersonal, die asymptomatisch mit MRSA kolonisiert sind und daher als Verbreitungsquelle unerkannt bleiben. Eine rechtzeitige Identifizierung solcher als Träger fungierenden Personen durch schnelle und einfach zu handhabende Verfahren kann die frühzeitige Einleitung geeigneter Hygienemaßnahmen gewährleisten und eine Ausbreitung der Stämme an Patienten unterbinden.

MRSA-Stämme lassen sich beispielsweise durch Rachen-, Nasen-, Wund- oder Perinealabstrich nachweisen. Die zum Nachweis der MRSA-Stämme verwendete Oligonukleotid-Sonde kann von einer oder von mehreren für MRSA spezifischen Sequenzen abgeleitet sein. DNA-Sequenzen, die spezifisch für die Identifizierung von MRSA-Stämmen eingesetzt werden können, sind dem Fachmann hinreichend bekannt. Erfindungsgemäß ist es besonders bevorzugt, dass die Oligonukleotid-Sonde von der Nukleotidsequenz des mecA-Gens abgeleitet ist, welches für das modifizierte Penicillin-Bindeprotein PBP2a kodiert (Song et al. (1987), FEBS Letters, 221: 167-171). Das mecA-Gen weist eine Größe von etwa 2,4 kb auf und kann bei verschiedenen Stämmen geringfügige Sequenzvariationen aufweisen. Die Oligonukleotid-Sonde, die im Rahmen der Erfindung Anwendung findet, ist vorzugsweise von der in SEQ ID NO:2 dargestellten Nukleotidsequenz des Gens mecA abgeleitet. Da auch weitgehend ungefährliche Organismen, wie beispielsweise verschiedene Stämme von *Staphylococcus epidermidis*, über ein mecA-Gen verfügen können, sollte im Rahmen eines Nachweises von MRSA in einem parallelen Ansatz das Vorliegen einer weiteren für *Staphylococcus aureus* spezifischen DNA-Sequenz untersucht werden. Bei dieser Sequenz kann es sich beispielsweise um die für bestimmte Pathogenitätsfaktoren von *Staphylococcus aureus* kodierende DNA handeln, z.B. um DNA, die für Protein A, Koagulase, Clumping Faktor, Exofoliatine A und B, oder ähnliche Pathogenitätsfaktoren kodiert. Darüber hinaus kann selbstverständlich auch eine für *S. epidermidis* spezifische Sequenz als Negativkontrolle verwendet werden.

Bei der Mischprobe, die die zu untersuchenden Zellen umfaßt, kann es sich ferner um Material aus lokalisierten Infektionsprozessen, wie beispielsweise aus Wunden, Abzessen und Läsionen, handeln. Dabei kann es sich beispielsweise um Abstriche oder Biopsate handeln. Bei geschlossenen Prozessen kann nach Desinfektion der Haut eine perkutane Punktion des Abszesses durchgeführt werden, und das durch Punktion gewonnene Material kann nach den erfindungsgemäßen Verfahren auf das Vorliegen bestimmter Erreger untersucht werden. Beispiele für bakterielle Erreger, die in Wunden nachgewiesen werden können, umfassen verschiedene Arten der Gattung Clostridium, die eine Myonekrose (Gasbrand) auslösen können. Dazu zählen beispielsweise *C. perfringens, C. septicum, C. histolyticum, C. novii* und *C. fallax.* In diesem Fall kann die Oligonukleotid-Sonde beispielsweise von der Nukleotidsequenz des Gens abgeleitet sein, das für das alpha-Toxin von *Clostridium perfringens* kodiert. Zahlreiche andere Beispiele für die Identifizierung von klinisch relevanten Bakterien werden sich für den Fachmann in naheliegender Weise aus der Beschreibung der vorliegenden Anmeldung ergeben.

Beim Nachweis von DNA-Sequenzen aus bakteriellen Erregern kann es sich bei der Probe, welche die zu untersuchenden Zellen umfaßt, beispielsweise auch um Liqour, Urin, Gelenkpunktat, Sputum, oder ähnliches handeln. Je nach Art und Herkunft der Probe und/oder der Art des nachzuweisenden bakteriellen Erregers kann es notwendig oder sinnvoll sein, vor Durchführung des erfindungsgemäßen Verfahrens zunächst eine Aufkonzentrierung des Zellmaterials durchzuführen. Sofern die biologische Probe, die die zu untersuchenden Zellen umfaßt, eine Flüssigkeit ist, kann die Aufkonzentrierung beispielsweise durch Filtration unter Verwendung eines Filters geeigneter Porengröße durchgeführt werden. Die Zellen können anschließend vom Filter direkt auf den Träger überführt werden.

Aufgrund der Struktur der bakteriellen Zellwand kann es insbesondere bei grampositiven Bakterien erforderlich sein, vor oder nach dem Aufbringen der Zellen auf den Träger eine Lyse oder zumindest eine partielle Lyse der Zellen durchzuführen, sodass die für das Bakterium spezifische DNA aus den Zellen freigesetzt wird. Eine Zelllyse kann z.B. durch Behandlung mit alkalischen Detergentien erreicht werden (Sambrook et al. (1989); Hrsg.; Molecular Cloning, 2. Auflage; Cold Spring Harbor Laboratory Press, New York). Bei bestimmten Bakterien, wie beispielsweise bei Bakterien der Gattung *Staphylococcus*, kann es sich als nützlich erweisen, zunächst einen enzymatischen Abbau der Bakterienzellwand durchzuführen. Geeignete Enzyme, die zu diesem Zweck eingesetzt werden können, umfassen beispielsweise Lysostaphin und Lysozym. Erfindungsgemäß ist es nicht relevant, ob die DNA-Zielsequenz in dem jeweiligen Bakterium als Plasmid oder als Bestandteil des bakteriellen Chromosoms vorliegt.

Gemäß einer weiteren Ausführungsform kann es sich bei der nachzuweisenden DNA-Sequenz um eine Nukleotidsequenz viralen Ursprungs handeln. In diesem Fall kann die nachzuweisende DNA-Sequenz episomal und/oder integriert in dem Genom der infizierten Zelle vorliegen. Für den Fachmann ist ersichtlich, dass bei Nachweis von viraler DNA im Rahmen des erfindungsgemäßen Verfahrens nur solche Zellen in Betracht kommen, die aus Bereichen bzw. Geweben des Körpers von Patienten erhalten wurden, welche von dem entsprechenden Virus infiziert sein könnten. Da Viren in der Regel eine Zell- oder Gewebespezifität aufweisen, und nicht beliebige Zellen des Körpers infizieren, richtet sich die Art der zu untersuchenden Zellen nach dem Virus, dessen DNA nachgewiesen werden soll. Bei den Viren kann es sich um solche handeln, die sich in das Genom der infizierten Zelle integrieren, oder um solche, die episomal in der Zelle vorliegen. Die DNA-Zielsequenz kann beispielsweise aus einem Virus stammen, das ausgewählt ist aus der Gruppe bestehend aus Humanes Immundefizienz Virus 1 und 2 (HIV-1 und HIV-2), Humanes T-Zell-Leukämie Virus 1 und 2 (HTLV-1 und HTLV-2), Respiratory-Syncytial-Virus (RSV), Adenovirus, Hepatitis B Virus (HBV), Hepatitis C Virus (HCV), Epstein-Barr-Virus (EBV), Humanes Papillomavirus (HPV), Varicella-Zoster-Virus (VZV), Cytomegalievirus (CMV), Herpes-simplex-Virus 1 und 2 (HSV-1 und HSV-2), Humanes Herpesvirus 8 (HHV-8, auch bekannt als Kaposi-Sarkom Herpesvirus) und Flaviviren, einschließlich Gelbfiebervirus, Dengue-Virus, Japanisches Enzephalitisvirus und West-Nil-Virus. Die vorliegende Erfindung ist jedoch nicht auf den Nachweis von DNA-Sequenzen aus den oben genannten Viren beschränkt, sondern kann problemlos auf andere veterinär- und/oder humanmedizinisch relevante Viren angewendet werden. Gemäß einer Ausführungsform ist die DNA-Zielsequenz eine Sequenz des humanen Papillomavirus, vorzugsweise eine Sequenz des humanen Papillomavirus 16 oder 18.

Humane Papillomaviren können episomal und/oder integriert in dem Genom der infizierten Zelle vorliegen vorliegen. Infektionen mit Papillomaviren treten in einer Vielzahl von Säugetieren auf, beispielsweise in Menschen, Schafen, Hunden, Katzen, Affen, Kaninchen und Rindern. Papillomaviren infizieren in der Regel Epithelzellen, was zu benignen epithelialen oder fibroepithelialen Tumoren an der Infektionsstelle führt. Papillomaviren weisen eine Wirtsspezifität auf und werden demgemäß in verschiedene Gruppen eingeteilt. Humane Papillomaviren (HPV) werden wiederum auf Basis ihrer DNA-Sequenzhomologie in mehr als 70 verschiedene Typen eingeteilt. Diese Typen verursachen verschiedene Erkrankungen. Die HPV-Typen 1, 2, 3, 4, 7, 10 sowie 26-29 verursachen gutartige Warzen. Die HPV-Typen 5, 8, 9, 12, 14, 15, 17 sowie 19-25 und 46-50 verursachen Läsionen in Patienten mit geschwächtem Immunsystem. Die Typen 6, 11, 34, 39, 41-44 und 51-55 verursachen gutartige Feigwarzen auf den Schleimhäuten des Genitalbereichs und des Respirationstrakts. Die HPV-Typen 16 und 18 sind von besonderem medizinischen Interesse, da sie epitheliale Displasien der Genitalschleimhaut verursachen und mit einem großen Teil der invasiven Karzinome von Cervix, Vagina, Vulva und Analkanal assoziiert sind. Die Integration der DNA des humanen Papillomavirus wird beim Cervix-Karzinom als entscheidend in der Karzinogenese angesehen. Humane Papillomaviren lassen sich beispielsweise über die DNA-Sequenz ihrer Kapsidproteine L1 und L2 nachweisen. Demgemäß eignet sich das Verfahren der vorliegenden Erfindung insbesondere für den Nachweis von DNA-Sequenzen der HPV-Typen 16 und/oder 18 in Gewebeproben, um das Risiko einer Karzinombildung einzuschätzen.

Die Träger, auf dem die zu untersuchenden Zellen aufgebracht werden, weisen erfindungsgemäß eine Oberfläche aus einem oder mehreren adsorbierenden Materialien auf, welche die zu untersuchenden Zellen sowie daraus freigesetzte Makromoleküle, insbesondere DNA-Moleküle, wie beispielsweise genomische DNA, auf ihrer Oberfläche immobilisieren können und damit verhindern, dass die Zellen und/oder die Makromoleküle sich bei Eintauchen des Trägers in eine Reaktionslösung von dem Träger ablösen. Grundsätzlich ist jedes Trägermaterial geeignet, auf dem die Zellen und die aus diesen freigesetzten Makromoleküle, insbesondere die genomischen DNA-Moleküle, für einen ausreichend langen Zeitraum immobilisieren werden, um die Hybridisierung der DNA-Zielsequenz und die anschließende Farbstoffbildung zu ermöglichen. Materialien, die sich zur Verwendung als Träger in dem erfindungsgemäß vorgeschlagenen Verfahren eignen, umfassen insbesondere poröse Materialien, wie beispielsweise Cellulose oder Cellulosederivate.

Der Träger kann beliebige Formen aufweisen, die an die jeweilige Verwendung des Trägers angepasst sind. Sofern beispielsweise die Entnahme von Mundschleimhautzellen beabsichtigt ist, kann der Träger beispielsweise in Form eines kleinen Bürstenkopfs ausgestaltet sein, der in geeigneter Weise für die Entnahme von Zellen, z.B. aus dem Nasen- und/oder Rachenraum, ausgestaltet ist. Solche Bürsten sind im Stand der Technik bekannt und umfassen u.a. die "Omni-Swab" Bürsten, die von der Firma Whatman GmbH (Dassel, Deutschland) vertrieben werden.

Ähnliche Produkte werden auch von anderen Anbietern (z.B. QIAgen, Baack Laborbedarf oder Isohelix Products) angeboten. Erfindungsgemäß wurde festgestellt, dass bei Abstrichen der Mundschleimhaut mittels einer Omni-Swab Bürste keine weiteren Schritte notwendig sind, um die partielle Lyse von humanen Mundschleimhautzellen zu bewirken. Vielmehr reicht bereits der Vorgang der Entnahme, d.h. das Reiben der Bürste entlang der Wangeninnenseite aus, um eine ausreichende Menge von Zellen der Mundschleimhaut auf der Bürste anhaften zu lassen und diese gleichzeitig zumindest teilweise aufzuschließen. Dabei werden auch die den Zellkern bildenden Membranen zerstört, sodass die genomische DNA der Zellen freigesetzt wird. Die bei diesem Vorgang auf die genomische DNA der Schleimhautzellen einwirkenden Scherkräfte sind groß genug, um einen Teil der DNA in einen fragmentierten, einzelsträngigen Zustand zu überführen, der die Hybridisierung mit einer erfindungsgemäß markierten Sonde ermöglicht. Es hat sich als zweckmäßig erwiesen, die Omni-Swab Bürste nach der Anwendung im Mundraum für 2-3 Minuten trocknen zu lassen, da dies eine noch stärkere Anhaftung des Zellmaterials und der DNA an das Material der Bürste bewirkt. Es ist selbstverständlich allerdings auch möglich, die zu untersuchenden Zellen mit herkömmlichen Mitteln zu entnehmen, die nicht in der Lage sind, die Zellen für die nachfolgenden Hybridisierungsschritte zu immobilisieren, und die Zellen anschließend auf einen geeigneten Träger aufzubringen. Beispielsweise können Mundschleimhautzellen zunächst durch einen Abstrich unter Verwendung herkömmlichen Wattestäbchen gewonnen werden, und in einem nachfolgenden Schritt auf einen Träger mit einer Celluloseoberfläche abgerieben werden.

Erfindungsgemäß muß zumindest ein Teil der Zellen in lysierter Form auf dem Träger vorliegen, um es der im nachfolgenden Schritt verwendeten Oligonukleotid-Sonde zu erlauben, mit der aus den Zellen freigesetzten DNA in Kontakt zu treten. Vorzugsweise liegt die DNA dabei in einzelsträngiger Form vor.

Wie bereits oben erwähnt, sind dem Fachmann verschiedene Verfahren zum Aufschluß von bakteriellen Zellen bekannt. Sofern es sich bei der DNA-Zielsequenz um eine Sequenz eines Säugetiers handelt, oder um die Sequenz eines Virus, das Zellen eines Säugetiers infiziert hat, sind die zu untersuchenden Zellen, aus denen die entsprechende DNA freigesetzt werden muß, tierischen Ursprungs. Dem Fachmann sind zahlreiche Verfahren bekannt, die sich zur Lyse tierischer Zellen, wie beispielsweise humaner Zellen, eignen. Im Gegensatz zu bakteriellen Zellen weisen tierische Zellen keine widerstandsfähige Zellwand auf und lassen sich daher leicht aufschließen. Als eukaryontische Zellen verfügen Säugetier-Zellen über einen Zellkern, der ebenfalls aufgeschlossen werden muss, damit es zur Freisetzung der genomischen DNA des Säugetiers kommen kann. Wie sich gezeigt hat ist in Fällen, in denen Abstriche der Mundschleimhaut durchgeführt werden, bereits die dadurch auf die Zellen ausgeübte mechanische Belastung ausreichend, um einen Teil der entnommenen Zellen zu lysieren und die genomische DNA aus den Zellkernen freizusetzen. In anderen Fällen kann es nützlich sein, vor oder nach dem Aufbringen der Zellen auf den Träger weitere Schritte durchzuführen, die eine (partielle) Lyse der Zellen fördern oder bewirken. Solche Maßnahmen sind dem Fachmann hinreichend bekannt und umfassen beispielsweise wiederholtes Einfrieren und Auftauen der die Zellen enthaltenden Probe, wiederholtes Aufnehmen und Ausstoßen einer die Zellen enthaltenen Lösung mittels einer Pipette, Zusatz von Detergentien und/oder weiterer die Integrität der Zellwand herabsetzender Mittel, sowie Erhitzen einer die Zellen enthaltenen Probe. Diese Maßnahmen führen nicht nur zur Zerstörung der Plasmamembran und des Zellkerns, sondern sorgen in der Regel auch gleichzeitig für die Erzeugung von einzelsträngigen DNA-Fragmenten, die in der nachfolgenden Hybridisierungsreaktion mit den markierten Sonden eine Bindung eingehen können.

Die auf dem Träger befindlichen Zellen werden anschließend mit mindestens einem Oligonukleotid in Kontakt gebracht, das in der Lage ist, mit der betreffenden DNA-Zielsequenz zu hybridisieren. Das mindestens eine Oligonukleotid weist zumindest in einem Abschnitt einen ausreichenden Grad an Komplementarität auf, um mit der ausgewählten DNA-Zielsequenz eine stabile Wechselwirkung durch komplementäre Basenpaarung einzugehen. Dieser als Hybridisierung bekannte Vorgang ist vorzugsweise spezifisch, d.h. das als Sonde verwendete Oligonukleotid hybridisiert unter stringenten Bedingungen nahezu ausschließlich mit der vorbestimmten DNA-Zielsequenz und bindet nicht im wesentlich Ausmaß an DNA-Sequenzen, die nicht komplementär oder nur unzureichend komplementär zu dem Oligonukleotid sind. Dem Fachmann ist bekannt, dass es bei jedem Hybridisierungsassay im gewissen Ausmaß zu einer unspezifischen Bindung der Sonde an die zu untersuchende DNA kommt. Es ist allerdings problemlos möglich, die Sequenz der Sonde sowie die übrigen Bedingungen der Hybridisierung derart zu wählen, dass eine unspezifische Bindung der Sonde weitgehend unterbleibt. Stringente Bedingungen, bei denen nahezu ausschließlich eine spezifische Hybridisierung erfolgt, können beispielsweise durch Verwendung eines Salzpuffers mit einer Salzkonzentration von 0,1 M bis 1,0 M Natrium- oder Kaliumionen und einem pH-Wert von etwa 7,0 bis 8,3 erzielt werden. Der Puffer kann ferner destabilisierende Mittel wie Formamid enthalten. Die Temperatur sollte zur Erreichung von stringenten Bedingungen etwa 3-8°C, vorzugsweise 5-6°C unter dem Schmelzpunkt des Oligonukleotids liegen. Der komplementäre Abschnitt des Oligonukleotids umfasst vorzugsweise etwa 5, 6, 7, 8, 9, 10, 11, 12, 13, 14, 15, 16, 17, 18, 19, 20, 22, 24, 26, 28, 30, 40 oder mehr Nukleotide. Der Abschnitt weist vorzugsweise über seine gesamte Länge eine Komplementarität zu einem entsprechenden Abschnitt in der DNA-Zielsequenz auf. Gemäß einer bevorzugten Ausführungsform ist das Oligonukleotid vollständig komplementär zu einem Bereich der DNA-Zielsequenz.

Wie vorliegend verwendet bezeichnet der Begriff "Oligonukleotid" ein Oligomer oder Polymer aus Nukleotid-Monomeren. Vorzugsweise liegt das Oligonukleotid als einzelsträngiges Molekül vor. Oligonukleotide lassen sich durch chemische Synthese oder durch PCR-Verfahren herstellen. Entsprechende Syntheseverfahren sind im Stand der Technik ausführlich beschrieben und umfassen beispielsweise das bekannte Phosphoramidit-Verfahren.

Der Begriff "Oligonukleotid" umfaßt wie vorliegend verwendet auch solche Oligomere oder Polymere, die neben oder anstelle der natürlich vorkommenden Basen Adenin, Guanin, Thymin, Cytosin und Uracil modifizierte Basen enthalten. Solche modifizierten Basen umfassen beispielsweise 5-Methylcytosin, 5-Hydroxymethylcytosin, Xanthin, Hypoxanthin, 2-Aminoadenin, 6-Methyladenin, 6-Methylguanin, 2-Thiouracil, 2-Thiothymin, 2-Thiocytosin, 5-Halouracil, 5-Halocytosin, 5-Propinyluracil, 5-Propinylcytosin, 6-Azouracil, 6-Azocytosin, 6-Azothymin, 5-Uracil, 4-Thiouracil, 8-Thioalkyladenin, 8-Hydroxyladenine, 8-Thioladenin, Thioalkylguanin, 8-Hydroxylguanin, 8-Thiolguanin, 7-Methylguanin, 7-Methyladenin, 8-Azaguanin und 8-Azaadenin, 7-Deazaguanin, 7-Deazaadenin, 3-Deazaguanin und/oder 3-Deazaadenin.

Vorzugsweise besteht das Oligonukleotid aus Nukleotidmonomeren, die über Phosphodiesterbrücken miteinander verbunden sind. Es können jedoch auch Oligomere oder Polymere verwendet werden, bei denen das Zucker-Phosphat-Rückgrat gegen funktionell ähnliche Strukturen ausgetauscht ist, beispielsweise gegen ein Peptid-Rückgrat oder gegen ein Phosphoramid-, Thiophosphat-, Mehtylphosphonat- oder Dithiophosphat-Rückgrat. Des Weiteren sind erfindungsgemäß auch Oligonukleotide umfaßt, die über einen oder mehrere modifizierte Zuckerreste verfügen. So können beispielsweise eine oder mehrere Hydroxylgruppen gegen Halogene oder aliphatische Gruppen ausgetauscht sein, oder sie können mit Ethern, Aminen oder ähnlichen Strukturen funktionalisiert sein.

Die Oligonukleotide können erfindungsgemäß jede beliebige Länge aufweisen. Besonders bevorzugt ist es, dass die Oligonukleotide eine Länge von etwa 6 bis 50 Nukleotiden aufweisen, beispielsweise eine Länge von 10, 12, 14, 16, 18, 20, 22, 24, 26, 28, 30, 32, 34, 36, 38, 40, 42, 44, 46, 48 oder 50 Nukleotiden. Die als Oligonukleotid-Sonden verwendeten Oligonukleotide können auch mehr als 50 Nukleotide umfassen, wie beispielsweise 60, 70, 80, 90 oder 100 Nukleotide. Die Länge und Zusammensetzung der Oligonukleotide richtet sich nach der Zusammensetzung der jeweiligen DNA-Zielsequenz und den Bedingungen des Hybridisierungsassays, wie z.B. die durch die verwendeten Puffer bedingte Ionenstärke usw. Diese Parameter sind dem Fachmann hinlänglich aus der Literatur bekannt.

Das als Sonde verwendete Oligonukleotid ist erfindungsgemäß mit einem beta-D-Galactopyranosid markiert. Als beta-D-Galactopyranosid werden vorliegend Moleküle bezeichnet, die aus einem Galactoserest und einem aglyconischen chromogenen Rest bestehen und über eine beta-glycosidische Bindung verknüpft sind. Die beta-glycosidische Bindung läßt sich durch Hydrolyse, z.B. durch enzymatische Hydrolyse, spalten. Bei der Hydrolyse entsteht ein wasserunlöslicher Farbstoff, der sich als Präzipitat absetzt. Im Stand der Technik sind verschiedene beta-D-Galactopyranoside beschrieben, die bei ihrer Hydrolyse unlösliche Farbstoffpräzipitate bilden. Diese beta-D-Galactopyranoside können im Rahmen der erfindungsgemäßen Verfahren zur Markierung der Oligonukleotid-Sonde verwendet werden.

Erfindungsgemäß ist es besonders bevorzugt, dass es sich bei dem beta-D-Galactopyranosid, das zur Markierung der Oligonukleotid-Sonde eingesetzt wird, um ein Indolyl-beta-D-galactopyranosid handelt. Als Indolyl-beta-D-galactopyranoside werden vorliegend Verbindungen bezeichnet, bei denen der Galactoserest über eine beta-glycosidische Bindung mit einem Indolylrest verknüpft ist. Der Indolylrest bildet den chromogenen Rest, der für die Farbstoffbildung verantwortlich ist. Diese bevorzugten beta-D-Galactopyranoside weisen folglich die nachstehende allgemeine Struktur auf.

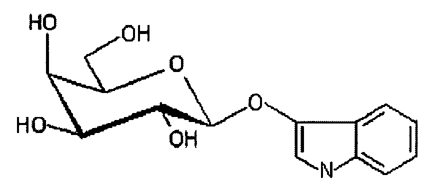

Der Indolylrest kann an einer oder an mehreren Stellen des Ringsystems substituiert sein. Beispielsweise können eine oder mehrere Wasserstoffatome der C-H Bindungen der heterozyklischen Indolyl-Gruppe mit einem Halogenatom, wie beispielsweise einem Chlor-, Brom- oder Iod-Atom, substituiert sein. Ferner kann auch der am Indol-Stickstoff gebundene Wasserstoff substituiert sein, beispielsweise gegen eine Alkylgruppe, wie beispielsweise Methyl, Ethyl oder Propyl.

Gemäß einer besonders bevorzugten Ausführungsform ist das Indolyl-beta-D-galactopyranosid 5-Brom-4-chlor-3-indoxyl-beta-D-galactopyranosid, das unter dem Namen x-Gal von verschiedenen Herstellern angeboten wird (z.B. Fermentas, St. Leon-Rot, Deutschland; Invitrogen, Karlsruhe, Deutschland). 5-Brom-4-chlor-3-indoxyl-beta-D-galactopyranosid ist durch seine Verwendung bei der sogenannten Blau-Weiß-Selektion hinreichend bekannt. Das farbloses x-Gal läßt sich durch die Aktivität einer beta-Galactosidase hydrolytisch zu 5-Brom-4-chlor-Indoxyl spalten. Das 5-Brom-4-chlor-Indoxyl wird in Gegenwart des Luftsauerstoffs zu einem wasserunlöslichen, blauen Farbstoff oxidiert (5,5'-Dibrom-4,4'-dichlorindigo).

Andere Indolyl-beta-D-galactopyranoside, die eine zu x-Gal vergleichbare Struktur aufweisen, umfassen beispielsweise N-Methylindolyl-beta-D-galactopyranosid, 5-Iod-3-indolyl-beta-D-galactopyranosid, 5-Brom-6-Chlor-3-indolyl-beta-D-galactopyranosid und 6-Chlor-3-Indoxyl-beta-D-galactopyranosid. Diese Verbindungen werden beispielsweise von GENTAUR-BIOXYS, Brüssel, Belgien, vertrieben. Das Galactopyranosid N-Methylindolyl-beta-D-galactopyranosid, das von GENTAUR-BIOXYS unter dem Namen Green-b-D-Gal erhältlich ist, bildet bei Hydrolyse ein grünes Farbstoffpräzipitat. Das Galactopyranosid 5-Iod-3-indolyl-beta-D-galactopyranosid, das unter dem Namen Purple-b-D-Gal von GENTAUR-BIOXYS erhältlich ist, bildet bei Hydrolyse ein violettes Farbstoffpräzipitat. Das unter dem Namen Red-b-D-Gal von GENTAUR-BIOXYS vertriebene 5-Brom-6-Chlor-3-indolyl-beta-D-galactopyranosid führt zu einem roten Präzipitat, während als Rose-b-D-Gal erhältliche 6-Chlor-3-Indoxyl-beta-D-galactopyranosid ein pinkes Präzipitat erzeugt.

Neben Indolyl-beta-D-galactopyranosiden sind dem auf dem Gebiet tätigen Fachmann auch andere beta-D-Galactopyranoside bekannt, die bei ihrer Hydrolyse nachweisbare wasserunlösliche, nicht-diffundierende Komplexe ausbilden. Solche Verbindungen umfassen beispielsweise Cyclohexenoesculetin-beta-D-galactopyranosid und 8-Hydroxyquinoline-beta-D-galactopyranosid, die in Gegenwart von Eisenionen schwarze, unlösliche Komplexe bilden. Die Herstellung dieser Verbindungen ist im Stand der Technik beschrieben (James et al., Appl. and Env. Microbiol. (1996), 62, Seiten 3868-3870). Ein weiteres Beispiel für eine Verbindung, die zur Verwendung in den erfindungsgemäßen Verfahren in Betracht kommt, ist para-Naphtholbenzein-beta-D-galactopyranosid, das bei der hydrolytischer Spaltung der glycosidischen Bindung einen unlöslichen pinken Farbstoff bildet (James et al., Appl. and Env. Microbiol. (2000), 66, Seiten 5521-5523). Es ist verständlich, dass Verbindungen, die sich durch den Austausch einzelner Gruppen oder Substituenten von den oben genannten beta-D-Galactopyranosiden unterscheiden, ebenfalls von der vorliegenden Erfindung umfaßt sind.

Die Markierung der Oligonukleotid-Sonden mit dem beta-D-Galactopyranosid erfolgt durch Kopplung des entsprechenden beta-D-Galactopyranosid an das Oligonukleotid. Verfahren der Kopplung sind im Stand der Technik beschrieben worden und liegen im Bereich des durchschnittlichen Könnens des Fachmanns. Erfindungsgemäß ist es besonders bevorzugt, dass die Kopplung zwischen dem Phosphatrest eines Nukleotids und einer Hydroxylgruppe im Zuckerring des beta-D-Galactopyranosid durchgeführt wird. Die Kopplung wird darüber hinaus von verschiedenen kommerziellen Anbieter durchgeführt (SEQLAB, Göttingen, Deutschland; MWG Biotech AG, Ebersberg, Deutschland; Metabion International AG, Planegg-Martinsried, Deutschland).

Das mindestens eine Oligonukleotid wird im Schritt c) des erfindungsgemäßen Verfahrens unter Bedingungen inkubiert, die eine Hybridisierung des mindestens einen Oligonukleotids an die DNA-Zielsequenz ermöglichen. Die Bedingungen, die einen Einfluß auf die Hybridisierung von komplementären DNA-Sequenzen ausüben, umfassen u.a. die Wahl der Ionenstärke in dem verwendeten Reaktionsansatz, die Temperatur, bei der die Lösung während der Hybridisierungsreaktion inkubiert wird, sowie der Gehalt an Substanzen, die mit der Ausbildung der Wechselwirkungen zwischen den komplementären DNA-Sequenzen interferieren können (z.B. Detergentien usw.). Der Fachmann kann die zu verwendende Sonde und die Reaktionsbedingungen problemlos aufeinander abstimmen. Die entsprechenden Parameter sind ausführlich in der Literatur beschrieben (Sambrook et al. (1989); Hrsg.; Molecular Cloning, 2. Auflage; Cold Spring Harbor Laboratory Press, New York). Die Inkubation kann für einen Zeitraum von wenigen Minuten, z.B. 5-15 Minuten, oder bis zu mehreren Stunden (z.B. 12-18 h erfolgen).

Es ist insbesondere wichtig, dass während der Hybridisierung der Oligonukleotid-Sonde an die DNA-Zielsequenz eine geeignete Temperatur gewählt wird. Dies bedeutet, dass der Reaktionsansatz bei einer Temperatur inkubiert werden sollte, die hoch genug ist, um eine unspezifische Bindung des mindestens einen Oligonukleotids an die DNA der Zellen auf dem Träger zu verhindern. Anderseits darf die Temperatur die Schmelztemperatur des Oligonukleotids nicht überschreiten, da ansonsten keine Bindung des Oligonukleotids an die vorbestimmte DNA-Zielsequenz erfolgen kann. Die Schmelztemperatur Tm eines Oligonukleotids ist (bei einer definierten Ionenstärke, pH-Wert und DNA-Konzentration) als die Temperatur definiert, bei der 50% des Oligonukleotids im Equilibrium an die DNA-Zielsequenz hybridisiert sind. Die Berechnung der Schmelztemperatur ist dem Fachmann bekannt und kann dazu verwendet werden, den optimalen Temperaturbereich für das Verfahren festzulegen. Sie kann näherungsweise durch die Formel Tm = (A+T) x 2°C + (G+C)x 4°C abgeschätzt werden. Wie vorliegend verwendet ergibt sich die Schmelztemperatur der im Rahmen der Erfindung verwendeten Oligonukleotide aus den zur Zielsequenz komplementären Bereichen der Oligonukleotide. Dies bedeutet, dass nur die Bereiche, in denen eine komplementäre Basenpaarung mit der Zielsequenz ausgebildet wird, für die Berechnung der Schmelztemperatur herangezogen werden. Da im Rahmen der erfindungsgemäßen Verfahren auch Oligonukleotide verwendet werden können, die Bereiche aufweisen, in denen keine Komplementarität zur DNA-Zielsequenz herrscht, ist es wichtig, dass lediglich die Bereiche der Oligonukleotide für die Berechnung verwendet werden, die mit entsprechend komplementären Bereichen aus der Zielsequenz hybridisieren. Maßgebend für den Tm-Wert sind ferner die Konzentration monovalenter Kationen (z.B. Na+) sowie die Konzentration von Doppelstrang-destabilisierender Mitteln (z.B. Formamid).

Wie bereits erwähnt wurde im Rahmen der vorliegenden Erfindung festgestellt, dass es nicht erforderlich ist, nach der Durchführung der Hybridisierungsreaktion zwischen der Oligonukleotid-Sonde und der DNA-Zielsequenz die gebundenen Oligonukleotid-Sonden von den ungebundenen Oligonukleotid-Sonden zu trennen. Die ungebundenen Oligonukleotide führen erst nach einer erheblichen Verzögerung von bis zu 15 Minuten zu einer Anfärbung der Reaktionslösung. Im Gegensatz dazu verursachen die an die DNA der Zellen gebundenen Oligonukleotid eine unmittelbare Farbreaktion. Demgemäß ist erfindungsgemäß möglich, die Schritte (b) bis (d) des Verfahrens nacheinander im selben Reaktionsansatz durchzuführen, ohne dass Zwischenschritte, wie z.B. eine Abtrennung von ungebundener Sonde, notwendig sind.

Der Nachweis des in Schritt d) gebildeten Hybridisierungsprodukts, d.h. des DNA-Duplexes aus markiertem Oligonukleotid und DNA-Zielsequenz, erfolgt durch Hydrolyse der glycosidischen Bindung des beta-D-Galactopyranosids, welches an das Oligonukleotid gekoppelt ist. Die Hydrolyse wird vorzugsweise durch ein Polypeptid katalysiert, das eine beta-Galactosidaseaktivität aufweist. Das Polypeptid kann dem Reaktionsansatz nach Abschluß des Hybridisierungsschritts zugegeben werden oder zu diesem Zeitpunkt im Reaktionsansatz freigesetzt werden. Als beta-Galactosidaseaktivität wird vorliegend eine enzymatische Aktivität verstanden, die in der Lage ist, eine beta-glycosidische Bindung zwischen einem Galactoserest und einem damit verknüpften aglyconischen Rest zu spalten. Vorzugsweise ist die beta-glycosidische Bindung eine solche, die das Kohlenstoffatom 1 des Galactoserests betrifft (wie z.B. die beta 1-4-glycosidische Bindung in Lactose). Die Fähigkeit eines Polypeptids, die beta-glycosidische Bindung zwischen einem Galactoserest und einem damit verknüpften aglyconischen Rest zu spalten, kann durch Inkubation des Polypeptids unter geeigneten Bedingungen (Puffer, pH-Wert, usw.) mit einem Substrat wie x-Gal überprüft werden.

Im Stand der Technik sind zahlreiche Enzyme mit beta-Galactosidaseaktivität aus prokaryontischen und eukaryontischen Organismen beschrieben worden. Gemäß einer besonders bevorzugten Ausführungsform ist das Polypeptid mit beta-Galactosidaseaktivität die beta-Galactosidase aus *Escherichia coli* oder eine Variante oder ein aktives Fragment derselben. Die Aminosäuresequenz der beta-Galactosidase aus *Escherichia coli* ist in SEQ ID NO:1 angegeben. Erfindungsgemäß sind ferner Varianten des in SEQ ID NO:1 gezeigten Polypeptids sowie enzymatisch aktive Fragmente des Polypeptids und seiner Varianten eingeschlossen. Unter Varianten eines Polypeptids werden solche Polypeptide verstanden, die sich durch einen oder mehrere Austausche von Aminosäuren von der Aminosäuresequenz des in SEQ ID NO:1 gezeigten Polypeptids unterscheiden. Bei dem Aminosäureaustausch kann es sich um einen konservativen oder nichtkonservativen Aminosäureaustausch handeln. Grundsätzlich kann jeder Aminosäurerest der in SEQ ID NO:1 gezeigten Aminosäuresequenz gegen eine andere Aminosäure ausgetauscht sein, sofern der Austausch nicht zu einem vollständigen Verlust der enzymatischen Aktivität führt. Im Allgemeinen werden Varianten des in SEQ ID NO:1 gezeigten Polypeptids eine signifikante Übereinstimmung mit der in SEQ ID NO:1 gezeigten Sequenz aufweisen. Vorzugsweise beträgt die Aminosäureidentität mehr als 60%, 70%, 80%, 90% oder mehr als 95%. Besonders bevorzugt beträgt die Aminosäureidentität mehr als 96%, 97%, 98% oder 99%.

Als Varianten gelten ferner auch solche Polypeptide, die sich von dem in SEQ ID NO:1 gezeigten Polypeptid durch ein oder mehrere zusätzliche Aminosäuren unterscheiden. Diese zusätzlichen Aminosäuren können sich innerhalb der in SEQ ID NO:1 gezeigten Sequenz befinden (Insertion) oder können an einen oder an beide Termini des Polypeptids angehängt sein. Insertionen können prinzipiell an jeder Position des in SEQ ID NO:1 gezeigten Polypeptids erfolgen, sofern der Austausch nicht zu einem vollständigen Verlust der enzymatischen Aktivität des Polypeptids führt. Besonders bevorzugt sind Varianten, bei denen die eine oder mehrere zusätzliche Aminosäure am C-Terminus und/oder N-Terminus angehängt sind. Somit umfaßt der Begriff Varianten auch Fusionspolypeptide, bei denen das in SEQ ID NO:1 gezeigte Polypeptid mit flankierenden Sequenzen fusioniert ist, die eine Aufreinigung des Proteins bei heterologer Expression erlauben. Beispiele für solche Sequenzen umfassen Histidinmodule, wie beispielsweise den 6xHis-tag, die über die Affinität zu immobilisierten Nickel-Ionen eine Aufreinigung des Fusionspolypeptids erlauben, oder Domänen des Proteins A, einem bakteriellen Zellwandprotein aus *Staphylococcus aureus* mit einer spezifischen Aktivität zur Fc-Region von Immunglobulinen der G-Klasse (IgG). Andere flankierende Sequenzen, die zur Aufreinigung von Fusionspolypeptiden verwendet werden können sind dem Fachmann hinlänglich bekannt.

Ferner gelten auch Polypeptide als Varianten des in SEQ ID NO:1 gezeigten Polypeptids, bei denen eine oder mehrere Aminosäuren im Vergleich zu dem in SEQ ID NO:1 gezeigten Polypeptid fehlen. Solche Deletionen können jede Aminosäure-Position der Sequenz von SEQ ID NO:1 betreffen, sofern der Austausch nicht zu einem vollständigen Verlust der enzymatischen Aktivität führt.

Die vorliegende Erfindung umfasst ferner enzymatisch aktive Fragmente des in SEQ ID NO:1 gezeigten Polypeptids und seiner wie oben definierten Varianten. Als Fragmente gelten im Rahmen der vorliegenden Erfindung solche Peptide oder Polypeptide, die sich von dem in SEQ ID NO:1 gezeigten Polypeptid und seinen Varianten durch das Fehlen einer oder mehrerer Aminosäuren am N-Terminus und/oder am C-Terminus des Peptids oder Polypeptids unterscheiden, wobei zumindest ein Teil der enzymatischen Aktivität erhalten bleibt.

Derivate des in SEQ ID NO:1 gezeigten Polypeptids oder seiner Varianten sind ebenfalls erfindungsgemäß umfasst. Derivate bezeichnen vorliegend Polypeptide, die gegenüber dem in SEQ ID NO:1 gezeigten Polypeptid oder seiner Varianten strukturelle Modifikationen, wie beispielsweise modifizierte Aminosäuren, aufweisen. Bei diesen modifizierten Aminosäuren kann es sich erfindungsgemäß um Aminosäuren handeln, die entweder durch natürliche Prozesse, wie beispielsweise Prozessierung oder posttranslationale Modifikationen, oder durch im Stand der Technik hinreichend bekannte chemische Modifikationsverfahren verändert wurden. Typische Modifikationen, denen die Aminosäuren eines der erfindungsgemäßen Polypeptide unterworfen werden können, umfassen Phosphorylierung, Glykosylierung, Acetylierung, Acylierung, Verzweigung, ADP-Ribosylierung, Quervernetzung, Disulfidbrückenbildung, Formylierung, Hydroxylierung, Carboxylierung, Methylierung, Demethylierung, Amidierung, Zyklisierung und/oder kovalente oder nicht-kovalente Bindung an Phosphotidylinositol, Lipide oder Flavin. Solche Modifikationen sind in der einschlägigen Literatur vielfach beschrieben, z.B. in Proteins: Structure and Molecular Properties, T. Creighton, 2. Auflage, W. H. Freeman and Company, New York (1993).

Es ist bevorzugt, dass die Varianten oder Derivate des in SEQ ID NO:1 gezeigten Polypeptids bzw. die enzymatisch aktiven Fragmente dieses Polypeptids bis zu 75 %, vorzugsweise bis zu 80%, 85%, 90%, 95% oder sogar bis zu 99% der Aktivität des in SEQ ID NO:1 gezeigten Polypeptids aufweisen.

Neben der spezifisch angeführten beta-Galactosidase aus *Escherichia coli* können beliebige andere prokaryontische und eukaryontische Enzyme mit beta-Galactosidaseaktivität im Rahmen der erfindungsgemäß offenbarten Verfahren verwendet werden. Im Stand der Technik sind zahlreiche beta-Galactosidasen bekannt, die aus prokaryontischen Organismen isoliert wurden. Beispiele für bakterielle beta-Galactosidasen umfassen beispielsweise solche, die aus *Bacillus cereus, Bacillus halodurans, Bacillus megaterium, Bacillus subtilis, Bacillus circulans, Lactococcus lactis, Lactobacillus acidophilus, Lactobacillus delbrueckii, Streptomyces coelicolor, Bacteroides fragilis, Yersinia pseudotuberculosis, Yersinia pestis, Pseudoalteromonas atlantica, Erwinia carotovora, Thermus thermophilus, Xanthomonas campestris, Alicyclobacillus acidocaldarius, Lactobacillus salivarius, Alteromonas macleodii, Erythrobacter litoralis, Clostridium perfringens, Clostridium beijerincki, Vibrio splendidus, Enterobacter cloacae, Enterococcus faecium, Vibrio vulnificus, Sulfolobus solfataricus, Bifidobacterium adolescentis, Streptococcus pneumoniae, Thermoanaerobacter mathranii, Pyrococcus woesei, Deinococcus geothermalis, Thermotoga maritima, Pyrococcus abyssi, Pyrococcus furiosus, Caldicellulosiruptor saccharolyticus, Streptococcus suis* oder *Xylella fastidiosa* isoliert wurden.

Ferner können auch beta-Galactosidasen pflanzlicher Herkunft verwendet werden, z.B. solche, die aus *Arabidopsis thaliana, Oryza sativa, Carica papaya, Asparagus officinalis, Capsicum annuum, Mangifera indica, Lycopersicon esculentum, Cicer arietinum, Brassica oleracea, Raphanus sativus, Prunus persica* oder *Sandersonia aurantiaca* isoliert und hinsichtlich ihrer Sequenz beschrieben wurden. Geeignete Enzyme aus Pilzen umfassen die beta-Galactosidasen aus *Penicillium canescens, Aspergillus niger, Aspergillus fumigatus, Aspergillus phoenicis, Hypocrea jecorina* oder *Thermomyces lanuginosus.*

Es können darüber hinaus auch beta-Galactosidasen aus tierischen Organismen verwendet werden, insbesondere solche, die aus Säugetieren, wie beispielsweise aus Geweben von Hunden, Ratten, Mäusen oder Primaten isoliert wurden. Die oben aufgeführten beta-Galactosidasen können aus den betreffenden Organismen isoliert oder rekombinant in anderen Organismen exprimiert werden.

Selbstverständlich können auch aktive Fragmente, Varianten oder Derivate dieser beta-Galactosidasen verwendet werden, wobei die Begriffe aktive Fragmente, Varianten und Derivate analog zu den oben in Bezug auf die SEQ ID NO:1 aufgeführten Definitionen zu verstehen sind.

Das zur Spaltung der glycosidischen Bindung verwendete Polypeptid kann neben der beta-Galactosidaseaktivität auch noch über eine weitere enzymatische Funktion verfügen, z.B. im Falle eines bifunktionalen Fusionspolypeptids (siehe z.B. Bulow, Eur J Biochem (1987), Vol 163, Seiten 443-448).

Das Polypeptid mit beta-Galactosidaseaktivität wird dem Reaktionsansatz zugesetzt oder in diesem freigesetzt, nachdem man dem mindestens einem Oligonukleotid ausreichend Zeit gegeben hat, mit der DNA-Zielsequenz aus den Zellen zu hybridisieren. Dies kann beispielsweise dadurch erreicht werden, dass das entsprechende enzymatisch aktive Polypeptid (ggf. in einem geeignet konzentrierten Puffer) in den Reaktionsansatz pipettiert wird. Gemäß einer besonderen Ausführungsform der vorliegenden Erfindung wird das enzymatisch aktive Polypeptid in Form von Wachskügelchen in den Reaktionsansatz eingebracht, wobei das Wachs eine Schmelztemperatur aufweist, die oberhalb der für die Hybridisierung angelegten Temperatur liegt. Somit erfolgt keine Freisetzung des enzymatisch aktiven Polypeptids während des Schritts der Hybridisierung. Nach erfolgter Hybridisierung der Sonden an die DNA- Zielsequenz wird die Temperatur des Ansatzes erhöht, sodass es zum Schmelzen der Wachskügelchen kommt und die enzymatisch aktiven Polypeptide freigesetzt werden. In diesem Schritt muß darauf geachtet werden, dass die Temperatur, die zum Schmelzen der Wachskügelchen führt, nicht so hoch ist, dass die Schmelztemperatur des markierten Oligonukleotids überschritten wird, da sich ansonsten das Oligonukleotid in Folge der hohen Temperatur von seiner Zielsequenz ablösen würde.

Nach erfolgter hydrolytischer Spaltung der glycosidischen Bindung des beta-D-Galactopyranosids kommt es zur Bildung und zur Präzipitation des wasserunlöslichen Farbstoffs. Der Farbstoff setzt sich im Bereich lipophiler Strukturen, wie z.B. im Bereich der auf dem Träger befindlichen lysierten Zellen, Zellmembranen, usw. ab, wodurch diese Bereiche auf dem Träger gefärbt werden. Die Färbung kann durch Sichtprüfung nachgewiesen werden. Die Färbung zeigt das Vorliegen der DNA-Zielsequenz in den auf dem Träger aufgebrachten Zellen an.

Die oben beschriebenen Verfahren lassen sich insbesondere zur Identifizierung eines unbekannten Nukleotids an einer vorbestimmten Position einer ansonsten bekannten DNA-Sequenz einsetzen, wie beispielsweise beim Nachweis eines Einzelnukleotid-Polymorphismus in der chromosomalen DNA von Menschen und nicht-menschlichen Säugetieren. Einzelnukleotid-Polymorphismen ("single nucleotide polymorphism"; SNP) stellen die häufigsten bei Säugetieren vorkommenden Sequenzvariationen dar. Bei einem Einzelnukleotid-Polymorphismus kommt es zum Austausch eines einzelnen Nukleotids in einem bestimmten Abschnitt eines DNA-Moleküls (z.B. eines Gens). Im menschlichen Genom findet man etwa alle 1000 Basenpaare einen Einzelnukleotid-Polymorphismus. Sofern dieser Austausch im kodierenden Bereich eines Gens liegt, kann er einen Aminosäureaustausch im kodierten Polypeptid zur Folge haben. Somit kann der Austausch eines einzelnen Nukleotids zu schwerwiegenden physiologischen Konsequenzen für das entsprechende Individuum führen. Einzelnukleotid-Polymorphismen sind im Zusammenhang mit einer Reihe von Erkrankungen beschrieben worden, beispielsweise bei der Alzheimer Erkrankung, der zystischen Fibrose, der Mukoviszidose, der Duchenne-Muskeldystrophie, Chorea Huntington, der Sichelzellenanämie und der malignen Hyperthermie. Einzelnukleotid-Polymorphismen spielen darüber hinaus eine wichtige Rolle bei der allergischen Medikamentenreaktion (z.B. auf Carbamazepin) sowie beim Stevens-Johnson-Syndrom (toxisch epidermale Nekrolyse). Des weiteren ist die Ansprechbarkeit auf ovarielle Stimulation in der Reproduktionsmedizin sowie auf betaadrenerge Medikamente bei der chronisch obstruktiven Lungenerkrankung durch Einzelnukleotid-Polymorphismen beeinflußt.

Im Stand der Technik sind eine Reihe verschiedener Verfahren zum Nachweis von Einzelnukleotid-Polymorphismen bekannt. In der Regel werden Verfahren angewendet, die auf eine Amplifizierung des Sequenzbereichs der genomischen DNA des Patienten beruhen, der den Einzelnukleotid-Polymorphismen enthält. Eine elegante Möglichkeit zum Nachweis eines Einzelnukleotid-Polymorphismen wurde von Landegren und Hood in US 4,988,617 beschrieben. Dieses Verfahren ist in Fachkreisen als Ligations-Detektionsreaktion (LDR) bekannt geworden. Das Verfahren beruht auf der Hybridisierung von zwei Oligonukleotiden an eine DNA-Zielsequenz, wobei die beiden Oligonukleotide unmittelbar nebeneinander an die DNA-Zielsequenz hybridisieren und in einem nachfolgenden Schritt durch eine Ligase miteinander verbunden werden. Das durch die Ligation entstandene längere Oligonukleotid wird mittels PCR nachgewiesen, wobei es nur zur Bildung eines PCR-Produkts kommt, wenn eine Ligation der beiden einzelnen Oligonukleotide stattgefunden hat. Eine solche Ligation findet wiederum nur statt, wenn in den aneinandergrenzenden Endbereichen der Oligonukleotide eine vollständige Basenpaarung mit der DNA-Zielsequenz erfolgt. Sofern jedoch in diesem Bereich eines der Nukleotide keine Basenpaarung mit der DNA-Zielsequenz ausgebildet hat (beispielsweise aufgrund eines Einzelnukleotid-Polymorphismus in der DNA-Zielsequenz), kommt es nicht zu einer Ligation der Oligonukleotide und folglich kann kein PCR-Produkt amplifiziert werden. Auf Basis dieses Prinzips läßt sich durch Verwendung der erfindungsgemäß markierten Oligonukleotide ein Schnelltest gestalten, der innerhalb weniger Minuten Auskunft über das Vorliegen des jeweiligen Einzelnukleotid-Polymorphismus geben kann, ohne auf eine PCR oder andere aufwendige Nachweisverfahren zurückgreifen zu müssen.

Gemäß einer besonderen Ausführungsform der vorliegenden Erfindung wird somit ein in vitro-Verfahren zum Nachweis einer DNA-Zielsequenz aus einer Gruppe von DNA-Sequenzen, deren Mitglieder sich in genau einer vorbestimmten Nukleotid-Position voneinander unterscheiden, bereitgestellt. Das Verfahren umfaßt Schritte, bei denen man
(a) Zellen auf einem Träger bereitstellt, wobei mindestens ein Teil der Zellen lysiert ist;
(b) die auf dem Träger befindlichen Zellen mit einem ersten und einem zweiten Oligonukleotid in Kontakt bringt, von denen mindestens eines mit einem beta-D-Galactopyranosid markiert ist, das bei Hydrolyse der glycosidischen Bindung einen wasserunlöslichen Farbstoff bildet, und die so gewählt sind, dass
   (i) sie jeweils mit einem endständigen Abschnitt in der Weise an die DNA-Zielsequenz hybridisieren können, dass die terminalen Nukleotide der beiden endständigen Abschnitte unmittelbar benachbart zueinander angeordnet sind, wobei das Nukleotid an der vorbestimmten Position der DNA-Zielsequenz eine komplementäre Basenpaarung mit einem Nukleotid in dem endständigen Abschnitt des ersten Oligonukleotids ausbildet, und dass
   (ii) bei Hybridisierung der beiden endständigen Abschnitte an eine andere DNA-Sequenz aus der Gruppe von DNA-Sequenzen aufgrund der fehlenden komplementären Basenpaarung zwischen dem Nukleotid an der vorbestimmten Position der DNA-Sequenz und einem Nukleotid in dem endständigen Abschnitt des ersten Oligonukleotids das terminale Nukleotid des endständigen Abschnitts des ersten Oligonukleotids nicht an die DNA-Sequenz hybridisieren kann, sodass die terminalen Nukleotide der beiden endständigen Abschnitte nicht unmittelbar benachbart zueinander angeordnet sind;
(c) die beiden Oligonukleotide unter Bedingungen inkubiert, die eine Hybridisierung der beiden Oligonukleotide an die DNA-Zielsequenz ermöglichen;
(d) die beiden hybridisierten Oligonukleotide mit einem Ligationsmittel in Kontakt bringt, das diese nur dann zu einem Ligationsprodukt verbindet, wenn die beiden terminalen Nukleotide der endständigen Abschnitte unmittelbar benachbart zueinander angeordnet sind;
(e) den Reaktionsansatz auf eine Temperatur erwärmt, bei der sich markierte, nicht-ligierte Oligonukleotide von der DNA-Zielsequenz ablösen, und bei der das Ligationsprodukt an die DNA-Zielsequenz hybridisiert bleibt;
(f) das an die DNA-Zielsequenz hybridisierte Ligationsprodukt mittels Hydrolyse der glycosidischen Bindung des beta-D-Galactopyranosids nachweist;
wobei die Bildung eines Farbstoffs im Bereich der auf dem Träger befindlichen Zellen auf das Vorliegen der DNA-Zielsequenz in den Zellen hinweist.

Bei der nachzuweisenden DNA-Zielsequenz handelt es sich vorzugsweise um die Sequenz eines Gens oder eines Teils eines Gens. Gemäß einer besonders bevorzugten Ausführungsform handelt es sich um die Sequenz eines humanen Gens oder eines Teils desselben. Die DNA-Zielsequenz ist erfindungsgemäß vollständig oder teilweise bekannt. Dies bedeutet, dass zumindest der Bereich um die vorbestimmte Nukleotid-Position hinsichtlich seiner Nukleotid- bzw. Basenabfolge bekannt ist, sodass entsprechende Oligonukleotide für die Hybridisierung an diesen Bereich erstellt werden können. Das Verfahren dient der Unterscheidung der DNA-Zielsequenz von anderen DNA-Sequenzen, die sich in mindestens einer, vorzugsweise in genau einer vorbestimmten Nukleotid-Position (d.h. durch eine Base) von der DNA-Zielsequenz unterscheiden. Der Begriff "vorbestimmt" bedeutet in diesem Zusammenhang, dass die Position, an der sich die verschiedenen DNA-Sequenzen voneinander unterscheiden, hinsichtlich ihrer Lokalisierung in den DNA-Sequenzen bekannt ist. Die DNA-Zielsequenz hat vorzugsweise eine Länge von etwa 20-20000 Nukleotiden, wobei eine Länge von etwa 50, 60, 70, 80, 90, 100, 200, 300, 400, 500, 600, 1000, 5000 oder 10000 Nukleotiden besonders bevorzugt ist.

Bei den Mitglieder der Gruppe von DNA-Sequenzen kann es sich beispielsweise um verschiedene allelische Varianten eines humanen Gens handeln. Wird beispielsweise die Sequenz eines bestimmten humanen Gens betrachtet, das in der betreffenden codierenden Sequenz an einer bekannten Position eine einzelne variable Position aufweist, so sind theoretisch neben der Sequenz des Wildtyps drei weitere Sequenzvarianten denkbar, die jeweils ein Nukleotid mit einer anderen Base in dieser Position aufweisen. Dies bedeutet, dass die DNA-Zielsequenz und die drei Varianten-Sequenzen eine Gruppe bilden, deren Mitglieder sich jeweils genau an einer vorbestimmten Nukleotid-Position von den anderen Mitgliedern der Gruppe unterscheiden.

Als Ausgangspunkt des Verfahrens dienen wiederum Zellen, die auf einem Träger bereitstellt werden, wobei mindestens ein Teil der Zellen lysiert ist, sodass die DNA aus den Zellen freigesetzt worden ist und dem Träger anhaftet. Vorzugsweise liegt die DNA dabei in einzelsträngiger Form vor. Die Zellen stammen vorzugsweise von einem Säugetier, wie z.B. einem Menschen, wobei es nicht darauf ankommt, welche Zellen des Körpers für das Verfahren verwendet werden. Da sich das zu identifizierende Nukleotid (z.B. ein Einzelnukleotid-Polymorphismus) in der genomischen DNA jeder Körperzelle des Säugetiers nachweisen lassen sollte, können für das Verfahren solche Zellen ausgewählt werden, die besonders einfach zu lysieren sind und/oder die besonders leicht erhalten werden können. Es kann sich bei den Zellen um Schleimhautzellen handeln, vorzugsweise um humane Schleimhautzellen. Besonders bevorzugt sind humane Mundschleimhautzellen. Als Träger kommen die oben beschriebenen Materialien in Betracht, wobei die Verwendung von "Omni-Swab" Bürsten oder vergleichbarer Produkte besonders bevorzugt ist.

Die auf dem Träger bereitgestellten Zellen werden mit einem ersten und einem zweiten Oligonukleotid in Kontakt gebracht, wobei mindestens eines mit einem beta-D-Galactopyranosid markiert ist, das bei Hydrolyse der glycosidischen Bindung einen wasserunlöslichen Farbstoff bildet. Es ist jedoch gleicherma-ßen möglich, beide Oligonukleotide mit demselben beta-D-Galactopyranosid oder mit verschiedenen beta-D-Galactopyranosiden zu markieren. Dies führt aufgrund der höheren Anzahl von Markierungen zu einer Verstärkung des Signals. Die bei diesem Verfahren einsetzbaren beta-D-Galactopyranoside sind oben beschrieben worden. Gemäß einer bevorzugten Ausführungsform ist das beta-D-Galactopyranosid ausgewählt aus der Gruppe bestehend aus 5-Brom-4-chlor-3-indoxyl-beta-D-galactopyranosid (x-Gal), N-Methylindolyl-beta-D-galactopyranosid, 5-Iod-3-indolyl-beta-D-galactopyranosid, 5-Brom-6-Chlor-3-indolyl-beta-D-galactopyranosid, 6-Chlor-3-Indoxyl-beta-D-galactopyranosid, para-Naphtholbenzein-beta-D-galactopyranosid, Cyclohexenoesculetin-beta-D-galactopyranosid und 8-Hydroxyquinoline-beta-D-galactopyranosid. Gemäß einer besonders bevorzugten Ausführungsform ist das beta-D-Galactopyranosid 5-Brom-4-chlor-3-indoxyl-beta-D-galactopyranosid (x-Gal).

Die Oligonukleotide sind so gewählt, dass sie jeweils mit einem endständigen Abschnitt an die DNA-Zielsequenz hybridisieren können. Dies bedeutet, dass jedes der beiden Oligonukleotide mindestens einen endständigen Abschnitt aufweist, der ausreichend komplementär zu einem entsprechenden Abschnitt der DNA-Zielsequenz ist, um eine Hybridisierung an die DNA-Zielsequenz zu ermöglichen. Vorzugsweise ermöglicht der Abschnitt eine spezifische Hybridisierung, d.h. eine Hybridisierung lediglich an den vorgesehenen Abschnitt der DNA-Zielsequenz und nicht an andere Sequenzen, die keine oder nur geringfügige Ähnlichkeit zu der DNA-Zielsequenz aufweisen. Der zu der DNA-Zielsequenz komplementäre Abschnitt ist endständig, d.h. er umfaßt einen Terminus (entweder den 5'-Terminus oder den 3'-Terminus) des jeweiligen Oligonukleotids und erstreckt sich in Richtung des entgegengesetzten Terminus des selben Oligonukleotids. Es ist bevorzugt, dass der für die Hybridisierung an die Zielsequenz erforderliche endständige Abschnitt der Oligonukleotide mindestens 5, 6, 7, 8, 9, 10, 11, 12, 13, 14, 15, 16, 17, 18, 19, 20, 22, 24, 26, 28, 30, 40, 50 oder mehr Nukleotide lang ist. Es ist dabei nicht unbedingt notwendig, dass jedes Nukleotid in diesem Abschnitt komplementär zu dem entsprechenden Nukleotid in der DNA-Zielsequenz ist, solange der Abschnitt insgesamt hinreichend komplementär ist, um mit einer genügend hohen Spezifität an den vorbestimmten Abschnitt der DNA-Zielsequenz hybridisieren zu können. Es ist bevorzugt, dass der endständige Abschnitt der Oligonukleotide eine vollständige Komplementarität zu einem entsprechenden Bereich der DNA-Zielsequenz aufweist. Selbstverständlich können erfindungsgemäß auch Oligonukleotide verwendet werden, die vollständig komplementär zu der DNA-Zielsequenz sind. Gemäß einer besonders bevorzugten Ausführungsform ist somit das erste und/oder zweite Oligonukleotid über seine gesamte Länge komplementär zur DNA-Zielsequenz. Sofern die Oligonukleotide Abschnitte aufweisen, die keine Komplementarität zur Zielsequenz aufweisen (wie z.B. in Figur 1 dargestellt), können diese Abschnitte für die Markierung mit dem beta-D-Galactopyranosid verwendet werden. Die Markierung kann allerdings auch an beliebigen anderen Stellen der Oligonukleotide erfolgen.

Die endständige Abschnitte des ersten und zweiten Oligonukleotids hybridisieren unmittelbar nebeneinander an die DNA-Zielsequenz, d.h. nach Hybridisierung der endständigen Abschnitte an die DNA-Zielsequenz sind die beiden terminalen Nukleotide der endständigen Abschnitte unmittelbar benachbart zueinander angeordnet. Unmittelbar benachbart zueinander angeordnet sind die terminalen Nukleotide der endständigen Abschnitte, wenn sie an zwei unmittelbar benachbarte Nukleotide in der DNA-Zielsequenz hybridisieren. Die Hybridisierung der beiden terminalen Nukleotide der endständigen Abschnitte an jeweils dazu komplementäre Nukleotide in der DNA-Zielsequenz ist eine notwendige Voraussetzung für die anschließende Verknüpfung der beiden Oligonukleotide zu einem Ligationsprodukt. Nur in dieser unmittelbar benachbarten Anordnung befinden sich die beiden terminalen Nukleotide der aneinandergrenzenden endständigen Abschnitte der Oligonukleotide in einem ausreichend geringem Abstand zueinander, dass sie von einem Ligationsmittel, z.B. einer Ligase, verbunden werden können. Sofern eines der beiden terminalen Nukleotide (oder beide) nicht mit einem entsprechenden Nukleotid in der zu untersuchenden DNA-Sequenz gepaart sein sollte, wird keine Ligation der terminalen Nukleotide erfolgen.

Die Oligonukleotide sind ferner so gestaltet, dass das Nukleotid an der vorbestimmten Position in der DNA-Zielsequenz nach Hybridisierung des ersten Oligonukleotids einem Nukleotid in dem endständigen Abschnitt des ersten Oligonukleotids zugeordnet ist. Nur bei der Hybridisierung an die gesuchte Zielsequenz bildet sich zwischen diesem Nukleotid des ersten Oligonukleotids und der vorbestimmten Position in der DNA-Zielsequenz eine komplementäre Basenpaarung aus. Bei Bindung des ersten Oligonukleotids an eine andere DNA-Sequenz, die sich an der vorbestimmten Position von der DNA-Zielsequenz unterscheidet, wird an dieser Position keine komplementäre Basenpaarung ausgebildet. Diese Fehlpaarung bedingt, dass das terminale Nukleotid des endständigen Abschnitts nicht an die DNA-Sequenz hybridisieren kann. Folglich befinden sich die beiden endständigen Abschnitte des ersten und zweiten Oligonukleotids nach Hybridisierung an die DNA-Sequenz nicht in einer unmittelbar benachbarten Position zueinander, und es kann keine Ligation erfolgen.

Damit die Fehlpaarung an der vorbestimmten Position im Falle einer DNA-Sequenz, die sich von der DNA-Zielsequenz unterscheidet, wirksam die Hybridisierung des terminalen Nukleotids des endständigen Abschnitts des ersten Oligonukleotids verhindern kann, muß die vorbestimmte Position in der zu untersuchenden DNA-Sequenz einem Nukleotid in dem ersten Oligonukleotid zugeordnet sein, das sich in der Nähe des terminalen Nukleotids des endständigen Abschnitts befindet. Vorzugsweise ist das Nukleotid in dem endständigen Abschnitt des ersten Oligonukleotids, das der vorbestimmten Position in der zu untersuchenden DNA-Sequenz zugeordnet ist, das terminale Nukleotid des endständigen Abschnitts (vgl. Figur 1). Es ist jedoch darüber hinaus auch möglich, dass die vorbestimmte Position in der untersuchenden DNA-Sequenz einem Nukleotid in dem ersten Oligonukleotid zugeordnet ist, das eine, zwei oder drei Positionen vom terminalen Nukleotid des endständigen Abschnitts entfernt liegt, d.h. die vorbestimmte Position der zu untersuchenden DNA-Sequenz kann einem der letzten vier Nukleotide in dem endständigen Abschnitt des ersten Oligonukleotids zugeordnet sein. Auch eine Fehlpaarung an diesen Positionen hat zur Folge, dass das terminale Nukleotid in dem endständigen Abschnitt des erste Oligonukleotids nicht mehr an eine ansonsten komplementäre DNA-Sequenz hybridisieren kann.

Das Prinzip der Bindung der beiden Oligonukleotide ist in Figur 1 an einer beispielhaften Ausführungsform veranschaulicht. Eine Zielsequenz (1) weist eine vorbestimmte Nukleotid-Position (6) auf. Bei dieser Nukleotid-Position kann es sich beispielsweise um die Position eines Einzelnukleotid-Polymorphismus handeln. Die an die DNA-Zielsequenz (1) hybridisierenden Oligonukleotide (2) und (3) werden hinsichtlich ihrer Sequenz so aufeinander abgestimmt, dass das Oligonukleotid (2) einen endständigen Sequenzabschnitt (4) aufweist, der stromaufwärts (d.h., bezogen auf eine von 5' nach 3' verlaufende DNA-Zielsequenz: in 5'-Richtung von der vorbestimmten Nukleotid-Position (6) in der DNA-Zielsequenz) an die DNA-Zielsequenz hybridisiert. Das zweite Oligonukleotid (3) weist einen endständigen Sequenzabschnitt (5) auf, der stromabwärts (d.h., bezogen auf eine von 5' nach 3' verlaufende DNA-Zielsequenz: in 3'-Richtung von der vorbestimmten Nukleotid-Position (6) in der DNA-Zielsequenz) an die DNA-Zielsequenz hybridisiert. Da die beiden endständigen Sequenzabschnitte (4) und (5) im Bereich der Termini vollständig mit der DNA-Zielsequenz hybridisieren, sind die beiden terminalen Nukleotide der beiden endständigen Abschnitte (4) und (5) unmittelbar benachbart zueinander angeordnet, d.h. bezogen auf eine von 5' nach 3' verlaufende DNA-Zielsequenz grenzt das 5'-terminale Nukleotid des stromaufwärts von der vorbestimmten Nukleotid-Position (6) hybridisierenden Oligonukleotids an das 3'-terminale Nukleotid des stromabwärts von der vorbestimmten Nukleotid-Position (6) hybridisierenden Oligonukleotids.

Die Figur 1 zeigt eine Ausführungsform des erfindungsgemäßen Verfahrens, bei der die vorbestimmte Nukleotid-Position (6) in der DNA-Zielsequenz dem 5'-terminalen Nukleotid von Oligonukleotid (2) gegenübergestellt ist. In einer anderen Ausführungsform ist die vorbestimmte Nukleotid-Position (6) dem 3'-terminalen Nukleotid von Oligonukleotid (3) gegenübergestellt. Darüber hinaus sind Ausführungsformen möglich, bei denen die vorbestimmte Nukleotid-Position (6) dem zweit-, dritt- oder viertletzten Nukleotid in einem der endständigen Abschnitte (4) und (5) gegenübergestellt ist. Im gezeigten Fall erfolgt zwischen der vorbestimmten Nukleotid-Position (6) in der DNA-Zielsequenz und dem 5'-terminalen Nukleotid von Oligonukleotid (2) eine komplementäre Basenpaarung, sodass die beiden terminalen Nukleotide der beiden endständigen Abschnitte (4) und (5) unmittelbar benachbart zueinander angeordnet sind. Somit kann es im weiteren Verlauf des Verfahrens zur Bildung eines Ligationsprodukts aus den beiden Oligonukleotids und damit zu einer Farbstoffbildung bei Nachweis des Ligationsprodukts kommen.

In einem nächsten Schritt des Verfahrens wird den beiden Oligonukleotiden Gelegenheit gegeben, mit der DNA-Zielsequenz zu hybridisieren. Die Bedingungen, die eine Hybridisierung erlauben, hängen im wesentlichen von der Zusammensetzung der Oligonukleotide ab. Der Fachmann wird diese Bedingungen problemlos für jeden einzelnen Fall auswählen können. Eine spezifische Hybridisierung wird insbesondere bei Temperaturen auftreten, die hoch genug sind, um eine unspezifische Hybridisierung der beiden Oligonukleotide mit nicht komplementären Bereichen der genomischen DNA zu unterbinden. Andererseits darf die Temperatur nicht so hoch gewählt sein, dass die Oligonukleotide trotz hinreichend komplementärer Abschnitte nicht an die DNA-Zielsequenz binden können. Vorzugsweise wird die Temperatur beim Hybridisierungsschritt des Verfahrens derart gewählt, das sie etwa 5-10°C unterhalb der geringeren der beiden Schmelztemperaturen der Oligonukleotide liegt. Die Schmelztemperatur der Oligonukleotide kann durch den Fachmann problemlos errechnet werden, wobei lediglich die zur Zielsequenz komplementären Bereiche in die Berechnung eingehen. Die beiden Oligonukleotide werden vorzugsweise so gewählt, dass sie beide über eine ähnliche Schmelztemperatur verfügen. Dies ermöglicht eine weitgehend einheitliche Hybridisierung an die DNA-Zielsequenz.

In einem nachfolgenden Schritt werden die hybridisierten Oligonukleotide mit einem Ligationsmittel in Kontakt gebracht, das diese nur dann zu einem Ligationsprodukt verbindet, wenn die beiden terminalen Nukleotide der endständigen Abschnitte unmittelbar benachbart zueinander angeordnet sind. Dies bedeutet, dass nur im Falle einer komplementären Basenpaarung zwischen dem Nukleotid an der vorbestimmten Position in der DNA-Zielsequenz (z.B. der den Polymorphismus begründenden Position) und dem entsprechenden Nukleotid in dem endständigen Abschnitt des hybridisierten Oligonukleotids eine Ligation erfolgen kann, da ansonsten die beiden terminalen Nukleotide der Oligonukleotide nicht in ausreichender Nähe zueinander angeordnet sind, um eine Verknüpfung des 5'-terminalen Nukleotids des stromaufwärts hybridisierten Oligonukleotids mit dem 3'-terminalen Nukleotid des stromabwärts hybridisierten Oligonukleotids zu ermöglichen. Sofern das Nukleotid an der vorbestimmten Position in der untersuchten DNA-Sequenz aufgrund mangelnder Komplementarität zu dem entsprechenden Nukleotid in dem endständigen Abschnitt des hybridisierten Oligonukleotids keine Basenpaarung ausbildet, ist eine Ligation der beiden terminalen Nukleotide der beiden Oligonukleotide nicht möglich, da sich die 5'-terminale Phosphatgruppe des einen Oligonukleotids nicht nahe genug an der 3'-terminalen OH-Gruppe des anderen Oligonukleotids befindet, um von dem Ligationsmittel verknüpft zu werden. Eine solche Verknüpfung ist nur möglich, wenn die beiden terminalen Nukleotide der Oligonukleotide durch komplementäre Basenpaarung mit Nukleotiden der DNA-Zielsequenz in einer entsprechend sterisch günstigen Position gehalten werden.

Das Ligationsmittel ist vorzugsweise eine DNA-Ligase. Als DNA-Ligasen werden vorliegend Enzyme verstanden, die DNA-Stränge miteinander verknüpfen. Sie bilden eine Phosphodiesterbindung zwischen einem 5'-Phosphatrest des einen DNA-Strangs und einer OH-Gruppe des anderen DNA-Strangs aus. Bei der DNA-Ligase kann es sich um eine Ligase aus dem T4-Bakteriophagen handeln. Diese Ligase verwendet ATP als Cofaktor. Daneben können auch Ligasen aus thermophilen oder hyperthermophilen Bakterien Anwendung finden. Auch die Verwendung der DNA-Ligase aus E. coli ist im Rahmen der vorliegenden Erfindung möglich. Dieses Enzym verwendet NAD als Cofaktor. Gemäß einer besonders bevorzugten Ausführungsform ist die DNA-Ligase die T4-DNA-Ligase. Gemäß einer weiteren bevorzugten Ausführungsform handelt es sich bei der Ligase um eine thermostabile Ligase, wie sie beispielsweise von Barany, Proc. Nat. Acad. Sci. USA 88: 189-193 (1991), im Zusammenhang mit einem solchen Verfahren beschrieben wurde.

Der Reaktionsansatz wird anschließend auf eine Temperatur erwärmt, bei der sich markierte, nicht-ligierte Oligonukleotide von der DNA-Zielsequenz und/oder von den anderen DNA-Sequenzen der Gruppe ablösen, und bei der das gebildete Ligationsprodukt an der DNA-Zielsequenz hybridisiert bleibt. Dies bedeutet, dass die für diesen Schritt ausgewählte Temperatur oberhalb des Schmelzpunktes der markierten Oligonukleotide liegt. Sofern auch nicht markierte Oligonukleotide zur Anwendung kommen, können diese auch an der DNA-Zielsequenz hybridisiert bleiben, da sie keinen Einfluß auf die nachfolgende Farbreaktion haben. Vorzugsweise liegt die ausgewählte Temperatur oberhalb der Schmelzpunkte der beiden Oligonukleotide. Sofern keine Ligation der beiden Oligonukleotide zu einem längeren Ligationsprodukt stattgefunden hat, werden sich in diesem Fall die beiden einzelnen Oligonukleotide durch die Erwärmung des Reaktionsansatzes von der DNA-Zielsequenz ablösen. Sofern hingegen bei der Ligation aus den beiden Oligonukleotiden ein Ligationsprodukt entstanden ist, so weist dieses einen längeren zur DNA-Zielsequenz komplementären Bereich auf und folglich einen deutlich höheren Schmelzpunkt. Dadurch ist es möglich, die Temperatur in diesem Reaktionsschritt so einzustellen, dass zwar der Schmelzpunkt der beiden einzelnen Oligonukleotide überschritten wird, nicht hingegen der Schmelzpunkt des Ligationsproduktes. Das Ligationsprodukt bleibt somit an die DNA-Zielsequenz hybridisiert und kann im folgenden die Bildung des Farbstoffs im Bereich der auf den Träger aufgebrachten Zellen bewirken.

In einem letzten Schritt wird das Hybridisierungsprodukt mittels Hydrolyse der glycosidischen Bindung des beta-D-Galactopyranosids nachgewiesen. Wie bereits beschrieben, kann diese Farbreaktion durch Zugabe oder Freisetzung eines Polypeptids mit beta-D-Galactosidaseaktivität erreicht werden. Vorzugsweise handelt es sich bei dem Polypeptid um die beta-Galactosidase aus *Escherichia coli* gemäß SEQ ID NO:1 oder eine Variante oder ein aktives Fragment derselben. Gemäß einer besonders bevorzugten Ausführungsform der vorliegenden Erfindung kann die Freisetzung des enzymatisch aktiven Polypeptids erreicht werden, indem die Polypeptide, die dem Reaktionsansatz zugegeben werden, in Wachskügelchen eingeschlossen sind. Die Verfahren zur Herstellung und Verwendung dieser Wachskügelchen sind im Stand der Technik hinreichend bekannt, insbesondere im Zusammenhang mit sogenannten Hotstart-PCR-Verfahren. Geeignete Wachskügelchen, die sich zum Einschluß von enzymatisch aktiven Proteinen oder Polypeptiden eignen, sind beispielsweise in US Patent 5,413,924 beschrieben. Da eine Farbreaktion im Rahmen des obigen Verfahrens nur durch das hybridisierte Ligationsprodukt verursacht werden soll, muß die für das Aufschmelzen der Wachskügelchen erforderliche Temperatur so gewählt werden, dass ein Aufschmelzen erst in einem Temperaturbereich erfolgt, der oberhalb der Schmelzpunkte der beiden Oligonukleotide liegt. Dadurch wird erreicht, dass eine Farbreaktion erst initiiert wird, wenn sich die markierten nicht-ligierten Oligonukleotide bereits von der DNA-Zielsequenz abgelöst haben. Die Temperatur zum Aufschmelzen der Wachskügelchen wird ferner so gewählt, dass es nicht zu einer Ablösung des Ligationsprodukts von der DNA-Zielsequenz kommt, d.h. die Temperatur liegt unterhalb der Schmelztemperatur des Ligationsprodukts.

Die Bildung eines Farbstoffs im Bereich der auf dem Träger befindlichen Zellen weist unmittelbar auf das Vorliegen des Ligationsprodukts aus den beiden Oligonukleotiden hin. Da die Bildung des Ligationsprodukts eine komplementäre Basenpaarung an der vorbestimmten Nukleotid-Position in der DNA-Zielsequenz und dem dieser Position gegenübergestellten Nukleotid des ersten Oligonukleotids erfordert, läßt sich aus der Farbstoffbildung im Bereich der auf dem Träger befindlichen Zellen unmittelbar schließen, dass die vorbestimmte Nukleotid-Position in der DNA-Zielsequenz komplementär zu dem diesem gegenübergestellten Nukleotid im ersten Oligonukleotid ist. Sofern keine Farbstoffbildung beobachtet wird, kann das Verfahren mit einer DNA-Sequenz, die ein Nukleotid mit einer anderen Base an der vorbestimmten Position aufweist, wiederholt werden, bis eine Farbstoffbildung beobachtet wird.

Gemäß einer bevorzugten Ausführungsform der Erfindung ist die DNA-Zielsequenz von der Sequenz des Wildtyps eines Gens umfaßt. Gemäß einer weiteren bevorzugten Ausführungsform der Erfindung ist die DNA-Zielsequenz von der Sequenz der Variante eines Gens umfaßt, d.h. von einer Sequenz, die sich durch eine Punktmutation von der Sequenz des Wildtyps des Gens unterscheidet. Vorzugsweise entspricht die DNA-Zielsequenz der Wildtyp- bzw. Varianten-Sequenz eines Gens. So kann beispielsweise die DNA-Zielsequenz von dem Gen umfaßt sein, welches für das humane synaptische Vesikelprotein 2A kodiert. Das für das synaptische Vesikelprotein 2A (SV2A) kodierende Wildtyp-Gen weist die in SEQ ID NO:3 aufgeführte Sequenz auf. Alternativ dazu kann die DNA-Zielsequenz von der Variante des in SEQ ID NO:3 aufgeführten Gens umfaßt sein, die sich in Position 8348 vom Wildtyp unterscheidet.

Es wurde herausgefunden, dass das SV2A-Gen einen Einzelnukleotid-Polymorphismus in Intron 6 aufweist, der für die Ansprechbarkeit eines Patienten auf bestimmte Therapeutika entscheidend ist. Der Polymorphismus wurde ausführlich in der am 28. September 2006 eingereichten internationalen Patentanmeldung (PCT/EP2006/066832) beschrieben. Der Polymorphismus betrifft den Austausch von Guanin (Wildtyp) in Position 8348 von SEQ ID NO:3 gegen ein Adenin (Variante). Dieser Einzelnukleotid-Polymorphismus ist ein Prädiktor für das Ansprechen eines Patienten auf eine antiepileptische Medikation mit verschiedenen Substanzen, wie beispielsweise Levetiracetam (Keppra^{©}; erhältlich von UCB GmbH, Kerpen, Deutschland). Die Substanz Levetiracetam wird in EP 0 162 036 beschrieben. Es handelt sich um ein Ethylanalog von Piracetam.

Patienten, die die Base "A" anstelle der Base "G" in Position 8348 von SEQ ID NO:3 aufweisen, haben ein etwa 5-fach erhöhtes Risiko, nicht im klinisch signifikanten Ausmaß auf die genannte Substanz zu reagieren. Demgemäß ist die Bestimmung des Genotyps eines epileptischen Patienten von hohem Aussagewert im Hinblick auf eine Behandlung mit Levetiracetam. Die Verfahren der vorliegenden Erfindung erlauben bereits beim ersten Treffen zwischen Arzt und Patient eine Bestimmung, ob ein Patient mit hoher Wahrscheinlichkeit auf eine Behandlung mit Levetiracetam reagieren wird. Dieser Polymorphismus läßt sich mittels der im Rahmen der Erfindung beschriebenen Verfahren schnell und verläßlich nachweisen, wobei beispielsweise die in SEQ ID NO:4 und SEQ ID NO:5 beschriebenen Oligonukleotide verwendet werden können. Das in SEQ ID NO:4 dargestellte Oligonukleotid entspricht dabei dem ersten Oligonukleotid des erfindungsgemä-ßen Verfahrens, welches so an die Sequenz des SV2A-Gens hybridisiert, dass das 3'-terminale "T" unmittelbar der Position des möglichen Polymorphismus in Position 8348 von SEQ ID NO:3 gegenübergestellt ist. Das in SEQ ID NO:4 dargestellte Oligonukleotid hybridisiert somit mit einem Bereich der Sequenz von SEQ ID NO:3, der sich von Position 8349 bis Position 8361 erstreckt. Das in SEQ ID NO:5 dargestellte Oligonukleotid entspricht dem zweiten Oligonukleotid, das in 5'-Richtung von der Position 8348 von SEQ ID NO:3 hybridisiert. Es hybridisiert mit einem Bereich der Sequenz von SEQ ID NO: 3, der sich von Position 8338 bis Position 8347 erstreckt, sodass das 5'-terminale "G" unmittelbar an das terminale "T" des Oligonukleotids von SEQ ID NO: 4 angrenzt. Selbstverständlich können auch längere Oligonukleotide verwendet werden, die die Sequenzen von SEQ ID NO:4 und SEQ ID NO:5 umfassen.

Wie für den Fachmann verständlich ist, kann das erfindungsgemäße Verfahren, das im Rahmen des Nachweises eines Polymorphismus angewendet wird, derart ausgestaltet sein, dass die Bildung eines Ligationsprodukts (und damit eine Farbstoffbildung im Bereich der Zellen) nur dann stattfindet, wenn die DNA des untersuchten Individuums keinen Polymorphismus aufweist, sondern dem Wildtyp entspricht. In diesem Fall entspricht die DNA-Sequenz des Wildtyps der nachzuweisenden DNA-Zielsequenz, und das Nukleotid des jeweiligen Oligonukleotids, das der Position des Einzelnukleotid-Polymorphismus gegenübergestellt ist, wird so gewählt, dass es eine Base aufweist, die komplementär zur Base des Nukleotids ist, welches im Wildtyp an dieser Position vorkommt.

Selbstverständlich können die Oligonukleotide auch so gestaltet werden, dass bei Durchführung des Verfahrens eine Ligation der hybridisierten Oligonukleotide und eine Bildung des Farbstoffs nur stattfindet, wenn die im Rahmen des erfindungsgemä-ßen Verfahrens untersuchte genomische DNA eine vom Wildtyp abweichende Sequenz aufweist, wie beispielsweise die Sequenz einen Einzelnukleotid-Polymorphismus. In diesem Fall entspricht die DNA-Sequenz der Variante bzw. Mutante der nachzuweisenden DNA-Zielsequenz, und das Nukleotid des jeweiligen Oligonukleotids, das der Position des Einzelnukleotid-Polymorphismus gegenübergestellt ist, eine Base aufweisen, die komplementär zu der Base ist, die im Zuge des Polymorphismus an dieser Position beschrieben worden ist.

Aus den bereits erwähnten Gründen ist es erfindungsgemäß möglich, die Schritte (b) bis (f) des Verfahrens ohne Zwischenschritte nacheinander im selben Reaktionsansatz durchzuführen.

Gemäß eines weiteren Aspekts betrifft die Erfindung die Verwendung eines Oligonukleotids, das mit einem wie oben definierten beta-D-Galactopyranosid markiert ist, in DNA-Hybridisierungsverfahren. In diesen Verfahren wird das Oligonukleotid als Sonde verwendet. Bei dem beta-D-Galactopyranosid handelt es sich vorzugsweise um Galactopyranosid 5-Brom-4-chlor-3-indoxyl-beta-D-galactopyranosid (x-Gal).

Ferner stellt die vorliegende Erfindung Kits zur Durchführung der im Rahmen der Erfindung beschriebenen Verfahren bereit. Die Kits umfassen mindestens ein Oligonukleotid, das mit einem beta-D-Galactopyranosid markiert ist, welches bei Hydrolyse der glycosidischen Bindung einen wasserunlöslichen Farbstoff bildet. Die Kits können auch ein oder mehrere weitere auf diese Weise markierte Oligonukleotide umfassen. Bei dem beta-D-Galactopyranosid handelt es sich vorzugsweise um Galactopyranosid 5-Brom-4-chlor-3-indoxyl-beta-D-galactopyranosid (x-Gal). Ferner enthalten die Kits Puffer und andere Reagenzien, die für die Durchführung von Hybridisierungsverfahren verwendet werden können. Die Kits können beispielsweise eine beta-Galactosidase oder geeignete Träger, wie beispielsweise die oben beschriebenen Bürstchen enthalten. Sofern das Kit dem Nachweis einer DNA-Zielsequenz aus einer Gruppe von DNA-Sequenzen, deren Mitglieder sich in genau einer vorbestimmten Nukleotid-Position voneinander unterscheiden (z.B. dem Nachweis eines Einzelnukleotid-Polymorphismus), kann es ferner ein Ligationsmittel, wie beispielsweise eine T4-DNA-Ligase, sowie Oligonukleotide, die unmittelbar nebeneinander an eine bestimmte Zielsequenz hybridisieren, enthalten. Die Kits können darüber hinaus Instruktionen zur Durchführung der oben beschriebenen Nachweisverfahren enthalten.

### BEISPIELE

Das folgende Beispiel erläutert den Nachweis des Einzelnukleotid-Polymorphismus, der das synaptische Vesikelprotein 2A (SV2A) betrifft. Die folgende Reaktionslösung wurde in einem Reaktionsgefäß von 1,5 ml hergestellt:

| | |
|---|---|
| 40 U | Beta-Galactosidase (eingeschlossen in Wachskügelchen) |
| 20 U | T4-DNA-Ligase |
| 50 µM | ATP |
| 66 mM | Tris-HCl (pH 7,6) |
| 150 nM | NaCl |
| 10 % | PEG |
| 6, 6 mM | MgCl₂ |
| 10 mM | DTT |
| ad 1000 µl | A. dest |

Die folgenden Oligonukleotide wurden zugegeben:
Oligo 1: 5'-AACATCGGCCAGGT-3'
Oligo 2: 5' GCCTCAGCC-3'-X-Gal

Das 3'-terminale Nukleotid von Oligo 1 ist ein "T" auf, sodass eine Basenpaarung mit der genomischen DNA des Probanden an diesem Nukleotid nur stattfindet, wenn an der entsprechenden Position in der genomischen DNA (Position 8348 von SEQ ID NO:3) abweichend von der Sequenz des Wildtyps ein "A" vorliegt (anstelle eines "G"). Mundschleimhautzellen wurde einem Patienten mit Hilfe eines "Omni-Swab" Bürste (Whatman) entnommen. Nach Trocknen der Bürste an der Luft für 2-3 Minuten wurde der Bürstenkopf in das obige Reaktionsgefäß gegeben. Die Reaktionslösung wurde zunächst bei einer Temperatur von 22°C gehalten. Nach 3 Minuten wurde die Temperatur des Reaktionsansatzes mit Hilfe eines Thermoelements auf 32°C erhöht. Es wurde festgestellt, dass es unter den oben beschriebenen Bedingungen lediglich bei Probanden mit einem durch Sequenzierung bestätigten Einzelnukleotid-Polymorphismus im SV2A-Gen zu einer Farbstoffbildung am Zellmaterial des Bürstenkopfs ion der Reaktionslösung kommt. Die Bildung des blauen Farbstoffs ist bereits nach 5 Minuten im Bereich der Bürste deutlich zu erkennen. Nach etwa 15 Minuten kommt es Blaufärbung der gesamten Reaktionslösung.

## Patentansprüche

1. In vitro-Verfahren zum Nachweis einer DNA-Zielsequenz in Zellen, bei dem man
(a) die Zellen auf einem Träger bereitstellt, wobei mindestens ein Teil der Zellen lysiert ist;
(b) die auf dem Träger befindlichen Zellen mit mindestens einem Oligonukleotid in Kontakt bringt, das in der Lage ist, mit der DNA-Zielsequenz zu hybridisieren, wobei das Oligonukleotid mit einem beta-D-Galactopyranosid markiert ist, das bei Hydrolyse der glycosidischen Bindung einen wasserunlöslichen Farbstoff bildet;
(c) das mindestens eine Oligonukleotid unter Bedingungen inkubiert, die eine Hybridisierung des mindestens einen Oligonukleotids an die DNA-Zielsequenz ermöglichen;
(d) das Hybridisierungsprodukt mittels Hydrolyse der glycosidischen Bindung des beta-D-Galactopyranosids nachweist,
wobei die Bildung eines Farbstoffs im Bereich der auf dem Träger befindlichen Zellen auf das Vorliegen der DNA-Zielsequenz in den Zellen hinweist.

2. Verfahren nach Anspruch 1, bei dem das beta-D-Galactopyranosid ausgewählt ist aus der Gruppe bestehend aus 5-Brom-4-chlor-3-indoxyl-beta-D-galactopyranosid (x-Gal), N-Methylindolyl-beta-D-galactopyranosid, 5-Iod-3-indolyl-beta-D-galactopyranosid, 5-Brom-6-Chlor-3-indolyl-beta-D-galactopyranosid, 6-Chlor-3-Indoxyl-beta-D-galactopyranosid, para-Naphtholbenzein-beta-D-galactopyranosid, Cyclohexenoesculetin-beta-D-galactopyranosid und 8-Hydroxyquinoline-beta-D-galactopyranosid.

3. Verfahren nach Anspruch 2, bei dem das beta-D-Galactopyranosid 5-Brom-4-chlor-3-indoxyl-beta-D-galactopyranosid (x-Gal) ist.

4. Verfahren nach einem der Ansprüche 1 bis 3, bei dem die Hydrolyse der glycosidischen Bindung des beta-D-Galactopyranosids durch ein Polypeptid mit beta-Galactosidaseaktivität erfolgt.

5. Verfahren nach Anspruch 4, bei dem das Polypeptid mit beta-Galactosidaseaktivität die beta-Galactosidase aus *Escherichia coli* gemäß SEQ ID NO:1 oder eine Variante oder ein aktives Fragment derselben ist.

6. Verfahren nach einem der Ansprüche 1 bis 5, wobei die Zellen aus einem Abstrich stammen.

7. Verfahren nach Anspruch 6, bei dem es sich bei dem Abstrich um einen Abstrich der Mundschleimhaut handelt.

8. Verfahren nach einem der Ansprüche 1 bis 7, bei dem die nachzuweisenden DNA-Zielsequenz eine bakterielle DNA-Sequenz ist.

9. Verfahren nach Anspruch 8, bei dem die bakterielle DNA-Sequenz ein bakterielles Resistenz-Gen ist.

10. Verfahren nach Anspruch 9, bei dem das bakterielle Resistenz-Gen das mecA-Gen ist.

11. Verfahren nach einem der Ansprüche 1 bis 7, bei dem die nachzuweisenden DNA-Zielsequenz eine virale DNA-Sequenz ist.

12. Verfahren nach Anspruch 11, bei dem die virale DNA-Sequenz eine Sequenz eines humanen Papillomavirus ist.

13. Verfahren nach Anspruch 12, bei dem die virale DNA-Sequenz eine Sequenz des humanen Papillomavirus 16 oder 18 ist.

14. Verfahren nach einem der vorhergehenden Ansprüche, bei dem die Schritte (b) bis (d) nacheinander in einem Reaktionsansatz durchgeführt werden.

15. In vitro-Verfahren zum Nachweis einer DNA-Zielsequenz aus einer Gruppe von DNA-Sequenzen, deren Mitglieder sich in genau einer vorbestimmten Nukleotid-Position voneinander unterscheiden, bei dem man
(a) Zellen auf einem Träger bereitstellt, wobei mindestens ein Teil der Zellen lysiert ist;
(b) die auf dem Träger befindlichen Zellen mit einem ersten und einem zweiten Oligonukleotid in Kontakt bringt, von denen mindestens eines mit einem beta-D-Galactopyranosid markiert ist, das bei Hydrolyse der glycosidischen Bindung einen wasserunlöslichen Farbstoff bildet, und die so gewählt sind, dass
(i) sie jeweils mit einem endständigen Abschnitt in der Weise an die DNA-Zielsequenz hybridisieren können, dass die terminalen Nukleotide der beiden endständigen Abschnitte unmittelbar benachbart zueinander angeordnet sind, wobei das Nukleotid an der vorbestimmten Position der DNA-Zielsequenz eine komplementäre Basenpaarung mit einem Nukleotid in dem endständigen Abschnitt des ersten Oligonukleotids ausbildet, und dass
(ii) bei Hybridisierung der beiden endständigen Abschnitte an eine andere DNA-Sequenz aus der Gruppe von DNA-Sequenzen aufgrund der fehlenden komplementären Basenpaarung zwischen dem Nukleotid an der vorbestimmten Position der DNA-Sequenz und einem Nukleotid in dem endständigen Abschnitt des ersten Oligonukleotids das terminale Nukleotid des endständigen Abschnitts des ersten Oligonukleotids nicht an die DNA-Sequenz hybridisieren kann, sodass die terminalen Nukleotide der beiden endständigen Abschnitte nicht unmittelbar benachbart zueinander angeordnet sind;
(c) die beiden Oligonukleotide unter Bedingungen inkubiert, die eine Hybridisierung der beiden Oligonukleotide an die DNA-Zielsequenz ermöglichen;
(d) die beiden hybridisierten Oligonukleotide mit einem Ligationsmittel in Kontakt bringt, das diese nur dann zu einem Ligationsprodukt verbindet, wenn die beiden terminalen Nukleotide der endständigen Abschnitte unmittelbar benachbart zueinander angeordnet sind;
(e) den Reaktionsansatz auf eine Temperatur erwärmt, bei der sich markierte, nicht-ligierte Oligonukleotide von der DNA-Zielsequenz ablösen, und bei der das Ligationsprodukt an die DNA-Zielsequenz hybridisiert bleibt;
(f) das an die DNA-Zielsequenz hybridisierte Ligationsprodukt mittels Hydrolyse der glycosidischen Bindung des beta-D-Galactopyranosids nachweist;
wobei die Bildung eines Farbstoffs im Bereich der auf dem Träger befindlichen Zellen auf das Vorliegen der DNA-Zielsequenz in den Zellen hinweist.

16. Verfahren nach Anspruch 15, bei dem das beta-D-Galactopyranosid ausgewählt ist aus der Gruppe bestehend aus 5-Brom-4-chlor-3-indoxyl-beta-D-galactopyranosid (x-Gal), N-Methylindolyl-beta-D-galactopyranosid, 5-Iod-3-indolyl-beta-D-galactopyranosid, 5-Brom-6-Chlor-3-indolyl-beta-D-galactopyranosid, 6-Chlor-3-Indoxyl-beta-D-galactopyranosid, para-Naphtholbenzein-beta-D-galactopyranosid, Cyclohexenoesculetin-beta-D-galactopyranosid und 8-Hydroxyquinoline-beta-D-galactopyranosid.

17. Verfahren nach Anspruch 16, bei dem das beta-D-Galactopyranosid 5-Brom-4-chlor-3-indoxyl-beta-D-galactopyranosid (x-Gal) ist.

18. Verfahren nach einem der Ansprüche 15 bis 17, bei dem die Hydrolyse der glycosidischen Bindung des beta-D-Galactopyranosids durch ein Polypeptid mit beta-Galactosidaseaktivität erfolgt.

19. Verfahren nach Anspruch 18, bei dem das Polypeptid mit beta-Galactosidaseaktivität die beta-Galactosidase aus *Escherichia coli* gemäß SEQ ID NO:1 oder eine Variante oder ein aktives Fragment derselben ist.

20. Verfahren einem der Ansprüche 15 bis 19, bei dem die DNA-Zielsequenz von der Wildtyp- oder der Varianten-Sequenz eines Gens umfaßt ist.

21. Verfahren nach Anspruch 20, bei dem die DNA-Zielsequenz von dem für das synaptische Vesikelprotein 2A kodierenden Gen, das die in SEQ ID NO:3 aufgeführte Sequenz aufweist, oder von der Variante desselben umfaßt ist.

22. Verfahren nach Anspruch 21, bei dem die Oligonukleotide die in SEQ ID NO:4 und SEQ ID NO:5 aufgeführten Sequenzen aufweisen.

23. Verfahren nach einem der Ansprüche 15 bis 22, bei dem die Zellen aus einem Abstrich stammen.

24. Verfahren nach Anspruch 23, bei dem es sich bei dem Abstrich um einen Abstrich der Mundschleimhaut handelt.

25. Verwendung eines Oligonukleotids, das mit einem beta-D-Galactopyranosid markiert ist, welches bei Hydrolyse der glycosidischen Bindung einen wasserunlöslichen Farbstoff bildet, in DNA-Hybridisierungsverfahren.

26. Verwendung nach Anspruch 25, bei der das beta-D-Galactopyranosid 5-Brom-4-chlor-3-indoxyl-beta-D-galactopyranosid (x-Gal) ist.

27. Kit zur Durchführung eines Verfahrens nach den Ansprüchen 1-14 oder 15-22, das mindestens ein Oligonukleotid enthält, das mit einem beta-D-Galactopyranosid markiert ist, welches bei Hydrolyse der glycosidischen Bindung einen wasserunlöslichen Farbstoff bildet.

28. Kit nach Anspruch 27, wobei das beta-D-Galactopyranosid 5-Brom-4-chlor-3-indoxyl-beta-D-galactopyranosid (x-Gal) ist.
